# EUROPEAN PATENT APPLICATION

(11) **EP 1 354 895 A1**
(43) Date of publication of application: **22.10.2003**
(21) Application number: 02008771.4
(22) Date of filing: 18.04.2002
(51) Int. Cl.: C07K 14/82, G01N 33/48, A61K 48/00, A61K 38/00, C12Q 1/68

(54) **Neopeptides useful for detection and treatment of cancer**

(71) Applicant: MTM Laboratories AG, 69120 Heidelberg (DE)
(72) Inventor: Von Knebel Doeberitz, Magnus Chirurg. Universität., 69120 Heidelberg (DE); Gebert, Johannes, 69221 Heidelberg (DE); Linnebacher, Michael, 66578 Stennweiler (DE); Wörner, Stefan, 69115 Heidelberg (DE); Ridder, Rüdiger, 69198 Schriessheim (DE); Bork, Peer, 69117 Heidelberg (DE); Yuan, Y. R., 69118 Heidelberg (DE)
(74) Representative: Schüssler, Andrea, Dr.

(57) **Abstract**

The present invention relates to NEOPEPTIDES useful for the detection and treatment of disorders associated with frameshift mutations in coding microsatellite regions. The NEOPEPTIDES are applicable in cancers, especially of DNA mismatch repair deficient (MMR) sporadic tumours and HNPCC associated tumours. The NEOPEPTIDES may be used for detection of disorders and in therapy such as vaccination therapy. The NEOPEPTIDES thus are useful for detection and early detection of disorders such as cancers and may be used for prevention of cancers and for the curative treatment of cancers and precancers.

## Description

The present invention relates to compounds useful for the detection and treatment of disorders associated with frameshift mutations in coding microsatellite regions. The compounds are especially applicable in cancers, especially of DNA mismatch repair deficient (MMR) sporadic tumours and HNPCC associated tumours. The compounds may be used for detection of disorders and in therapy such as immuno-therapy . The compounds thus are useful for detection and early detection of disorders such as cancers and may be used for prevention of cancers and for the curative treatment of cancers and precancers.

Tumour cells accumulate mutations in components of cellular pathways, that are essential for the maintenance of normal growth and differentiation. In human epithelial tumours, 2 types of genetic instability have been identified: chromosomal instability (CIN), which marks structural and numerical chromosomal aberration in aneuploid neoplastic cells, and microsatellite instability (MSI), which reflects length variations at repetitive DNA sequences in tumour cells. The type and spectrum of mutated genes markedly differs among CIN and MSI tumours, suggesting distinct but not mutually exclusive pathways of carcinogenesis. MSI occurs in about 90% of hereditary nonpolyposis colorectal cancers (HNPCC) as well as in about 15% of sporadic tumours of the colon and other organs, and is caused by mutational inactivation of various DNA mismatch repair genes.

The accumulation of genetic alterations and resulting mutant proteins represent a major obstacle for tumour cells to escape immune surveillance. The mutant proteins or peptides encoded by expressed mutant genes may elicit a specific cellular immune response and thus may be recognized by CTL. This is especially true concerning mutations resulting from chromosomal instabilities, but also pertains to more subtle genetic alterations, such as small deletions and insertions in microsatellites.

This situation can be used for prevention and therapies in cancers. The cancer cells are characterized by the expression of neo-peptides, which arouse from the genetic alterations. These proteins are not present in normal, non cancerous tissues. Thus, the neo-peptides may be used to distinguish on a molecular level between tumours and normal tissues. As tools suitable for the molecular discrimination antigen specific molecules or cells may be used. Thus it is possible to elicit an immune response against frameshift peptides, as might arise from MSI mutations, specific for tumours. Using cytotoxic T lymphocytes or FS8-specific antibodies it is thus possible to specifically attack and eliminate tumour cells in organisms and tissues.

A large number of genes containing coding microsatellites have been identified. Various of these genes show mutations within the microsatellites with certain frequencies in sporadic tumours. For few of these genes it could be shown, that they are involved in the majority of cases of particular tumours. For example the (A)₁₀ tract within the TGFβRII gene and the (G)₈ tract within the BAX gene are commonly mutated in gastrointestinal cancers such as colon cancer or gastric cancer.

The T-cell mediated immune response on the other hand provides a strong and specific selection pressure for the rapidly growing tumour cells (Tomlinson I, et al., Nat. Med. 1999; **5**: 11-2). Thus tumour evolution is forced to circumvent the immune surveillance by the cellular immune response. As a consequence of this selection pressure, mutations in genes of the antigen processing or presenting machinery arise in MMR-deficient tumour cells. In fact evasion of the immune surveillance by acquiring β2-microglobulin mutations has been observed at high frequency in MSI⁺ tumour cells (Bicknell DC, et al., Curr. Biol 1996; **6:** 1695-7). Other targets of specific mutation or down-regulation of expression are TAP1/TAP2 or HLA alleles. Direct down-regulation of expression of immunogenic epitopes is also a possible mechanism of immune escape as has been shown for melanoma-associated antigens in vivo (Jager E, et al., Int. J. Cancer 1997; **71**: 142-7). Due to this fact the relevant epitopes may not be detectable by the immune system.

The immunogenic frameshift peptides known to be expressed by cells affected by disorders in association with microsatellite instability provide useful tools for the therapy and diagnosis of said diseases. Yet to broaden the spectrum of disorders, that may be addressed by the respective frameshift peptides, even more expression products, arising from frameshift mutations should be available.

The present invention provides compounds associated with microsatellite instability for the use in therapy and diagnosis of disorders such as cancer.

The present invention is based on the inventors' findings, that a number of genes harbouring microsatellite regions is mutated in particular disorders, and gives rise to the expression of novel frameshift peptides. The novel frameshift peptides arise from frameshift mutations in microsatellites in the coding regions of the genes CHD2, UVRAG, ELAVL3, TCF6L1, ABCF1, AIM2, TAF1B, MACS and HT001.

It is one aspect of the present invention to provide nucleic acid sequences of the genes CHD2, UVRAG, ELAVL3, TCF6L1, ABCF1, AIM2, TAF1B, MACS and HT001, that have frameshift mutations within their coding regions.

A second aspect of the present invention is to provide new frameshift peptides, that occur in a wide range of different MSI⁺ tumours.

A third aspect of the present invention is a method for detection of MSI⁺ tumours using said frameshift peptides as molecular markers.

A fourth aspect of the present invention is a method for treatment of MSI⁺ tumours using said frameshift peptides for therapeutic purposes.

The present invention thus provides compounds and methods for the therapy and detection of disorders associated with frameshift peptides arising from mutations in coding microsatellite regions. The diagnostic and therapeutic methods are suited for application in disorders such as tumours or in precursory stages of disorders.

Nucleic acid molecules according to the present invention may comprise polynucleotides or fragments thereof. Preferred polynucleotides may comprise at least 20 consecutive nucleotides, preferably at least 30 consecutive nucleotides and more preferably at least 45 consecutive nucleotides of the sequences. The nucleic acids according to the present invention may also be complementary or reverse complementary to any of said polynucleotides. Polynucleotides may for example include single-stranded (sense or antisense) or double-stranded molecules, and may be DNA (genomic, cDNA or synthetic) or RNA. RNA molecules comprise as well HnRNA (containing introns) as mRNA (not containing introns). According to the present invention the polynucleotides may also be linked to any other molecules, such as support materials or detection marker molecules, and may, but need not, contain additional coding or non-coding sequences.

Mutation as used in the context of the present invention may comprise insertions or deletions of one or several nucleotides (or nucleotide repeats) within the specified microsatellite sequences. In a preferred embodiment of the present invention the number of nucleotides to be inserted or deleted is not 3 or must not be divisible by 3, such that the mutation leads to a frameshift with respect to the translational reading frame of the downstream nucleic acid sequence. Thus the nucleic acid sequence downstream of the mutation point will render a polypeptide-sequence different from the native sequence encoded by the respective gene. The mutation in these cases leads to a novel peptide sequence (a neo-peptide). Commonly the new peptide sequence is short due to the fact, that novel stop codons arise from the shift in the reading frame.

Frameshift mutations in microsatellites are usually due to DNA polymerase slippage and may be characterized by the type of the repeat. Thus in mono nucleotides repeats this type of mutation renders 1nt insertion or deletion. In dinucleotide repeats and tetranucleotide repeats mutations are insertions or deletions of 2 or 4 nt respectively. For example commonly (-1) mutations occur in mononucleotide repeats (MNRs). In these mutations one nucleotide is deleted such that the reading frame is shifted by one nucleotide toward the 5' end of the gene compared to the original reading frame. This type of mutation renders a reading frame identical to that produced by (+2) mutations, which arise from two nucleotide insertions in the respective microsatellites. The respective (-1) or (+2) polypeptides might differ by one amino acid. The other frameshift mutation variant leading to frameshift mutations differing from (-1) mutations concerning the resulting reading frame is the (+1) mutation, arising from one nucleotide insertion, thus rendering a reading frame one nucleotide toward the 3' end of the gene compared to the original reading frame. This mutation type gives a reading frame identical to that of (-2) mutations, wherein the encoded polypeptides differ by one amino acid. (-3) and (+3) mutations are irrelevant according to the present invention, for they do not give rise to frameshift polypeptides.

Frameshift polypeptides as used herein shall comprise any polypeptides or fragments thereof, that arise by a frameshift mutation within a coding microsatellite sequence of a gene. A gene may harbour one or more coding microsatellite regions, that may give rise to frameshift peptides. The coding microsatellites according to the present invention comprise mononucleotide repeats, dinucleotide repeats, trinucleotide repeats, tetranucleotide repeats and pentanucleotide repeats of any length. According to present invention coding microsatellites contain preferably at least 3 and more preferably at least 5 repeats of the respective nucleotide pattern (1-5 nucleotides, which are repeated).

The frameshift polypeptides as used according to the present invention comprise at least 1, more preferred at least 2 and even more preferred at least 3 amino acids of the mutated part of the polypeptide. Additionally, the frameshift polypeptides may comprise fragments of the originally non-mutated proteins and/or may be fused to any other polypeptide sequences suitable for the purposes of the present invention. Examples of such polypeptide sequences are linker sequences, or structural peptide sequences, such as beta barrels, loop sequences etc. that facilitate the immunogenicity of the frameshift sequences according to the present invention within the fusion polypeptide.

Frameshift polypeptides suitable for the method disclosed herein are immunogenic polypeptides. This requires, that the polypeptides may stimulate immune responses in host organisms either in the form the polypeptides adopt in their natural environment and/or especially in the form the polypeptides adopt after processing by the cellular antigen processing and presenting machinery.

According to the present invention the frameshift polypeptides may also be represented by nucleic acids coding for said polypeptides. These nucleic acids may for example be used for the *in situ* expression of the respective polypeptides. For the purpose of expression of nucleic acids, the particular nucleic acids may be joined with suitable other nucleic acid sequences, that enable for the cloning and expression of said nucleic acids encoding the frameshift polypeptides.

In certain embodiments of the present invention frameshift polypeptides may comprise fusion or chimeric polypeptides containing sequences disclosed herein. Fusion proteins comprise the frameshift polypeptide according to the present invention together with any second and further polypeptides, such as e.g. one more frameshift polypeptide of the same sequence or of another sequence. Heterologous polypeptides may comprise enzymes, receptor molecules, antigens, antigenic or immunogenic epitopes or fragments, antibodies or fragments thereof, signalling polypeptides or signal transducing polypeptides, labelled polypeptides etc.. In one embodiment of the invention the fusion peptides may be constructed for enhanced detection or purification of the frameshift polypeptides, or of complexes of the frameshift polypeptides with the respective immunological entities according to the present invention. For the purpose of purification tags, such as e.g. his-tags, myc-tags etc. may be added to the polypeptides. For the purpose of detection antigenic portions, enzymes, chromogenic sequences etc. may be fused to the polypeptides. The fusion proteins of the present invention may (but need not) include a linker peptide between the first and second polypeptides.

A nucleic acid sequence encoding a fusion protein of the present invention is constructed using known recombinant DNA techniques to assemble separate nucleic acid sequences encoding the first and second polypeptides into an appropriate expression vector. The 3' end of a nucleic acid sequence encoding the first polypeptide is ligated, with or without a peptide linker, to the 5' end of a nucleic acid sequence encoding the second polypeptide ensuring the appropriate reading frames of the sequences to permit mRNA translation of the two nucleic acid sequences into a single fusion protein that retains the biological activity (antigenicity) of both the first and the second polypeptides.

A peptide linker sequence may be employed to separate the first and the second polypeptides by a distance sufficient to ensure, that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

Immunological entities as used in the context of the present invention shall comprise any components of the mammalian immune system, that are able to specifically react with an antigenic epitope. Such immunlogical entities may comprise for example antibodies, all immunoglobulins, such as e.g. IgG, IgM, IgA, IgE, IgD, specific CD8+ T-cells or specific T-helper cells.

Disorders associated with frameshift mutations according to the present invention comprise for example degenerative diseases, such as neurodegenerative diseases, vascular disorders, disorders caused by stress, such as oxidative stress, chemically induced stress, irradiation induced stress, etc. and cancers including all sporadic cancers as well as HNPCC associated cancers. Cancers as used herein may comprise e.g. colorectal cancer, small cell lung cancer, liver cancer (primary and secondary), renal cancer, melanoma, cancer of the brain, head and neck cancer, gastrointestinal cancers, leukemias, lymphomas. prostate cancer, breast cancer, ovary cancer, lung cancer, bladder cancer etc..

In one embodiment of the present invention the peptides disclosed herein may be used for the diagnosis of disorders associated with frameshift mutations in coding microsatellite regions.

Diagnosis as used in the context of the present invention may comprise determining the presence or absence and/or the level of frameshift peptides or of specific immunological entities directed against particular frameshift peptides in a sample, and assessing diagnosis from said presence or absence and/or level of frameshift peptides and/or immunological entities specifically directed against said frameshift polypeptides.

Based upon the determined presence or absence and/or the levels of frameshift peptides or of immunological entities specifically directed against particular frameshift peptides in the samples individuals can be subdivided into subgroups. The subgroups may be created according to scientific or clinical data, such as e.g. survival, recurrence of disease, frequency of metastases etc., related to the presence or absence and/or levels of frameshift peptides or of particular frameshift peptides in samples of tissues affected with a particular disorder, of tissues being in question of being affected with a particular disorder or of tissues at risk of being affected with a particular disorder.

Based upon these subgroups an assessment of prognosis may be done. According to the subgroups the therapy of the individuals affected by the disorders (e.g. tumours) may be tailored.

Monitoring may comprise detecting the presence or absence or level of frameshift peptides or of immunologic entities specifically directed against frameshift polypeptides in samples taken at different points in time and determining the changes in said levels or presences or absences. According to said changes the course of the disease can be followed. E.g. the occurrence of frameshift peptides or of immunologic entities directed against frameshift peptides, that have not been present at an earlier time-point may be indicative of the progression of evolution of the affected tissue. The course of the disease may be used to select therapy strategies for the particular individual.

Another aspect of diagnosis and monitoring of the disease course according to the present invention may comprise the detection of minimal residual disease. This may comprise for example the detection of presence and/or level of frameshift peptides or of immunologic entities specifically directed against said frameshift polypeptides, that have not been present in earlier examinations in one or more body samples following initial therapy of an individual once or at several timepoints. According to the presence and/or level of frameshift peptides or of immunologic entities specifically directed against new frameshift polypeptides detected in the samples one may select a suitable therapy for the particular individual.

Furthermore the diagnostic method maybe carried out to detect disseminated tumor cells in biological samples as MRD diagnosis of minimal residual disease. For this purpose the detection of the level of immunological entities or the presence of frameshift peptides or of immunological entities, specific for particular frameshift peptides, that have not been detected in prior examinations, may be performed.

A sample according to the method of the present invention may comprise any sample comprising frameshift peptides or immunological entities as defined above. Samples may comprise samples of clinical relevance, such as e.g. blood, serum, secretions, smears, body fluids, urine, semen, stool, bile, biopsies, cell- and tissue-samples. Biopsies as used in the context of the present invention may comprise e.g. resection samples of tumors, tissue samples prepared by endoscopic means or needle biopsies of organs.

Such samples may comprise for example intact cells, lysed cells or any liquids containing polypeptides, antibodies, immunoglobulins or cells specifically directed against frameshift peptides. Even solids, to which peptides, cells, cell fragments or antigen binding polypeptides, such as antibodies or immunoglobulins may adhere, or may be fixed to, may be samples according to the method disclosed herein. The method for detection of the level of the frameshift peptides or of the immunological entities according to the present invention is any method, which is suited to detect very small amounts of specific frameshift peptides or of specific immunological entities in biological samples. The detection reaction according to the present invention is a detection either on the level of polypeptides, nucleic acids, antibodies or on the level of cells specific for particular antigens.

The detection may be carried out in solution or using reagents fixed to a solid phase. Solid phases may comprise beads of a variety of materials, such as e.g. agarose, dextrane polymers, polystyrene, silica, etc. or surfaces of suitable materials such as e.g. polystyrene, glass, agarose, protein, dextran etc. coated surfaces etc.. The detection of one or more frameshift peptides or of one or more immunological entities, such as immunoglobulins or cells carrying specific antigen recognizing epitopes, with different antigen binding speificities may be performed in a single reaction mixture or in two or more separate reaction mixtures. Alternatively the detection reactions for several frameshift peptides or of several immunological entities may for example be performed simultaneously in multi-well reaction vessels.

Applicable formats for the detection reaction according to the present invention may be, (reverse) blotting techniques, such as Western-Blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as ELISA, RIA, Elispot assay, lateral flow assays, immuno-cytochemical methods etc..

For diagnostic purposes detection procedures related to one single frameshift polypeptides or to immunological entities specifically recognizing said frameshift peptides may be performed. Furthermore detection procedures may be performed that are tailored to display the presence or absence or the level of one or more sets of polypeptides or immunological entities directed against these polypeptides, wherein the sets have been put together according to rational combinatorial parameters as they are given below.

The method for detection of the level of the frameshift polypeptides according to the present invention is any method, which is suited to detect very small amounts of specific biologically active molecules in biological samples. The detection reaction according to the present invention is a detection either on the level of nucleic acids or on the level of polypeptides.

The detection may be carried out in solution or using reagents fixed to a solid phase. The detection of one or more molecular markers, such as polypeptides or nucleic acids, may be performed in a single reaction mixture or in two or separate reaction mixtures. Alternatively the detection reactions for several marker molecules may for example be performed simultaneously in multi-well reaction vessels. The markers characteristic for the frameshift polypeptides may be detected using reagents that specifically recognise these molecules. The detection reaction for the marker molecules may comprise one or more reactions with detecting agents either recognizing the initial marker molecules or recognizing the prior molecules used to recognize other molecules.

In one preferred embodiment of the invention the detection of the level of frameshift polypeptides is carried out by detection of the level of nucleic acids coding for the frameshift polypeptides or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognizing and binding to said nucleic acids. This method can be performed as well in vitro as directly in situ for example in the course of a detecting staining reaction. The use of this detection procedure is restricted to cases, where hybridisation properties of the respective frameshift mutations are significantly altered in comparison to the respective wild-type nucleic acids. Another way of detecting the frameshift polypeptides in a sample on the level of nucleic acids performed in the method according to the present invention may comprise an amplification reaction of nucleic acids. In these cases a subsequent reaction displaying the presence or absence of a frameshift mutation within the coding microsatellite region is necessary.

In another preferred embodiment of the invention the detection of the level of frameshift polypeptides is carried out by determining the level of expression of a protein. The determination of the frameshift polypeptides on the protein level can for example be carried out in a reaction comprising an antibody specific for the detection of the frameshift polypeptides. The antibodies can be used in many different detection techniques for example in western-blot, ELISA or immuno-precipitation. Generally antibody based detection can be carried out as well in vitro as directly in situ for example in the course of an immuno-histochemical staining reaction. Any other method for determining the amount of particular polypeptides in biological samples can be used according to the present invention.

Furthermore according to the present invention diagnosis may comprise detection of immunological entities specifically recognizing particular frameshift peptides using reagents that specifically recognise these immunological entities alone or in complex with their respective antigen (e.g. antibodies), or reagents, that are specifically recognized by the immunological entities themselves (e.g. the antigen). In one embodiment the antigen may be fused to another polypeptide, so as to allow binding of the antigen by the immunological entity in question and simultaneously binding of the second part of the fusion protein by another (labelled) antibody for the detection. The detection reaction for the immunological entities may comprise one or more reactions with detecting agents either recognizing the initial entities or recognizing the prior molecules used to recognize the immunological entities.

In one preferred embodiment of the invention the detection of the level of immunological entities specific for frameshift peptides is carried out on the level of antibodies. This may be e.g. performed using the specific interaction between the respective frameshift peptides with the antibodies. The determination of the presence or absence and/or the level of the antibodies may for example be carried out with recombinantly produced frameshift peptides. The peptides can be used in many different detection techniques for example in western-blot, ELISA or immuno-precipitation. In one embodiment the detection of antibodies is carryout as antibody capture assay (Antibodies A laboratory Manual, Harlow, Ed. et al., Cold Spring Harbor Laboratory 1988).

In another embodiment of the invention the detection of the specific antibodies is carried out using monoclonal or polyclonal antibodies specifically recognizing the antigen binding epitope of the first antibodies. For this purpose the above mentioned immunological detection procedures may be applied. In a further embodiment chimeric antigens may be employed in the detection reaction. Such chimeric antigens may for example comprise fusion proteins combining the antigenic epitope of a frameshift polypeptide, recognized by the antibody in question, fused to another antigen, that may be recognized by a detection antibody. The particular antigens within the chimeric polypeptide may be separated by a linker or spacer region.

Any other method for determining the amount of particular antibodies or immunoglobulins in biological samples can be used according to the present invention.

Generally the detection of the antibodies according to the present invention may be performed as well in vitro as directly *in situ* for example in the course of an immuno-histochemical or immuno-cytochemical staining reaction.

In another embodiment of the present invention cells exhibiting specificity for a particular antigen may be detected by any method suitable for that purpose known to those of ordinary skill in the art. Methods may for example comprise proliferation-assays, cytokine-ELISAs, ELISpot assays, intracellular FACS-staining, PCR-mediated identification of peptide-specific cytokine (or similar) -expressing cells, tetramer-staining, cytotoxicity assays and DTH-(delayed type hypersensitivity) reactions.

In case of proliferation-assays induction of peptide-specific T-cell proliferation may be measured by methods known to those of skill in the art. This can be achieved by simple counting of cells, by measuring incorporation of labelled nucleotides into cellular DNA or by measuring level and/or activity of cellular protein(s). Cytokine-ELISA may comprise identification of peptide-specific cytokine-secreting cells by measuring cytokine levels in supernatant. In the course of an ELISpot assay the number of peptide-specific cytokine (i.e. IFN-g)- secreting cells in a sample is determined. Similarly the intracellular FACS-staining identifies cytokine-expressing cells on the protein level. In contrast (real-time) PCR may be used for identification of peptide-specific cytokine (or similar) -expressing cells on the transcript level, In the course of a tetramer-staining assay the label is a tetramer-molecule of recombinant MHC-class I molecules, loaded with specific peptide and coupled to a dye. The tetramer binds to the T-cell receptor. Cytotoxicity assays are a method for identification of cells, that can recognize and kill target cells in a peptide-specific manner. DTH-(delayed type hypersensitivity) reaction is based on the measuring of skin-reaction of vaccinated persons after intradermal (or similar) application of peptide(s).

The detection reaction further may comprise a reporter reaction indicating the presence or absence and/or the level of the frameshift polypeptides or of the immunological entities. The reporter reaction may be for example a reaction producing a coloured compound, a bioluminescence reaction, a chemiluminescent reaction, a fluorescence reaction, generally a radiation emitting reaction etc.. The detection reactions may for example employ antibodies or binding reagents that are detectably labelled.

Furthermore the binding of detection molecules to the peptides or immunological entities in question may be detected by any measurable changes in physical or physical-chemical properties, such as changes in spectroscopic properties, in magnetic resonance properties etc.. Different polypeptides or immunological entities or immunological entities of different specificities may be recognized by different methods or agents. This may be due to difficulties in detection of several entities, or entities with particular specificities by a particular method. An advantage of the use of different detection techniques for different polypeptides and/or immunological entities or for immunological entities with different specificities may for example be, that the different reporter signals referring to different immunological entities could be distinguished.

Generally in a method according to the present invention the detection of different polypeptides, of different immunological entities such as the detection of immunoglobulins and the detection of immunocompetent cells may be performed simoultaneously.

For all detection purposes optionally the original sample may be concentrated by any suitable means known to those of ordinary skill in the art. Furthermore steps may be involved to selectively extract polypeptides and/or immunological entities from the sample mixture such as affinity based purification techniques either employing specific antibodies or the respective antigen recognized by the entities in question.

In another aspect the method according to the present invention may be used for the treatment of disorders associated with frameshift mutations within coding microsatellite regions of genes.

The frameshift peptides disclosed herein may be used in vaccines, that may comprise one or more frameshift polypeptides.

The compositions and methods according to the present invention may be applied to any eukaryotic organisms exhibiting an immunologic defense system. The eukaryotic organisms are for example animals of agricultural value such as pigs, cows, sheep, etc., companion animals, such as cats, dogs, horses etc., animals employed in research purposes such as mice, rats, rabbits, hamsters etc. or humans.

According to the present invention frameshift polypeptides that comprise an immunogenic portion may be used for immuno-therapy for the treatment of cancer. Immunotherapy may be broadly classified into either active or passive immunotherapy. In active immunotherapy, treatment relies on the in vivo stimulation of the endogenous host immune system to react against tumours with the administration of immune response-modifying agents (for example, tumour vaccines, bacterial adjuvants, and/or cytokines). A patient may be afflicted with disease, or may be free of detectable disease. Accordingly, the compounds disclosed herein may be used to treat cancer or to inhibit the development of cancer. The compounds are preferably administered either prior to or following primary treatment of tumours such as surgical removal of the tumours, treatment by administration of radiotherapy and/or conventional chemotherapeutic drugs or any other mode of treatment of the respective cancer or its precursors.

In passive immunotherapy, treatment involves the delivery of biologic reagents with established tumour-immune reactivity (such as effector cells or antibodies) that can directly or indirectly mediate antitumour effects and does not necessarily depend on an intact host immune system. Examples of effector cells include T lymphocytes (for example, CD8+ cytotoxic T-lymphocytes, CD4+ T-helper, tumour-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive transfer immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T-cell cultures in order to generate sufficient number of cells for immunotherapy. In particular, antigen-presenting cells, such as dendritic-, macrophage- or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T-cells: Principles Revisited," Immunological Reviews, 157:177, 1997).

According to the present invention sets of frameshift polypeptides may be employed to generate and/or isolate tumour-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8+ CTL or CD4+ T-helper cells clones may be isolated from the patient, expanded using standard tissue culture techniques, and returned to the patient.

Alternatively, peptides corresponding to immunogenic portions of the polypeptides of the invention may be employed to generate tumour reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CeIIPro Incorporated's (Bothell, Wash.) CEPRATE.TM. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumour antigen-specific T-cells is then expanded using standard techniques and the cells are administered back to the patient.

In another embodiment, T-cell receptors and/or antibodies specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-expressing and/or presenting dendritic cells may either be transferred into a patient, or employed to stimulate T-cells to provide antigen-specific T-cells which may, in turn, be administered to a patient. The use of peptide-pulsed dendritic cells to generate antigen-specific T-cells and the subsequent use of such antigen-specific T-cells to eradicate tumours in a murine model has been demonstrated by Cheever et al, (Immunological Reviews, 157:177, 1997).

In another preferred embodiment of the present invention a set of frameshift polypeptides is used, that comprises at least 5, more preferably at least 7 and most preferred at least 10 frameshift polypeptides. Due to immunological as well as practical concerns the set of frameshift peptides used as a vaccine may not include anunlimited number of frameshift peptides. Preferably the set of frameshift peptides comprises at maximum 15, in a more preferred embodiment at maximum 20 and in the most preferred embodiment of the invention at maximum 30 frameshift polypeptides. In order to ensure a maximum range of disorders to be addressed by the selected set of frameshift polypeptides the members of the set have to be selected by reasonable considerations. The frameshift polypeptides to be used as a set according to the present invention are selected with respect to a number of parameters characterizing said polypeptides.

One crucial aspect influencing the selection of the frameshift peptides is the mutation frequency of the relevant microsatellite region and thus the frequency of a particular expressed frameshift polypeptide. Mutation frequency as used in the context of the present invention pertains to the percentage of samples within a defined range of total samples, which show a particular mutation. Any method suitable for the determination of the percentage of individuals in a range of samples displaying the existence of a particular genotype or phenotype (with respect to the expression of polypeptides) may be employed for the determination of the frequency according to the present invention. The frequencies may be determined e.g. as described below in example 1.

The frequency may be the frequency of frameshift mutation within a coding microsatellite region or a frequency of expression of a frameshift polypeptide. Furthermore the frequency may be e.g. a frequency over a total of different tumour entities, the frequency with respect to a particular tumour entity, a frequency over several tumours entities restricted to particular stages of tumourigenesis or a frequency with respect to a particular tumour entity restricted to particular stages of tumourigenesis.

The frequency according to the present invention has to be determined taking into account a range of samples sufficient to render significant data. For example preferably at least 50 to 100 tumours may be included in the range of samples analyzed. In case, that a smaller number of samples has been taken into account for determination of the frequency, a variation of the determined frequency may take place, if the range of samples is broadened.

Generally any frequency as determined may be used as a tool for the choice of a set of frameshift polypeptides according to the present invention. To ensure best results for the method according to the present invention a largest possible number of samples has to be taken into account. Yet if there are only rare data, a frequency may also be determined with respect to a restricted number of samples. This may especially be true, if the data for the restricted population of samples indicates a quite high frequency of a particular peptide, and thus implies a high therapeutic value for the respective peptide. Especially in cases, where a frequency is to be determined related to a particular tumour entity or related to particular stages of the tumourigenesis, the population of samples may be restricted.

The mutation may occur at any time during the tumour evolution and may be persistent or may be eliminated from the genome. Thus the frequency may comprise the frequency of the expression of a peptide at a particular stage of tumourigenesis or the occurrence of a genetic mutation at a particular defined stage of tumourigenesis. In one embodiment of the invention the frequency for the mutation is determined taking into account the widest possible range of samples. In this embodiment the method disclosed herein may be especially useful for the preventive vaccination of tumours. In another embodiment of the present invention the frequencies of mutation are related to specified tumour entities. In this embodiment the method according to the present invention may e.g. especially be useful for immuno-therapeutic approaches in treatment of tumours or in the preventive vaccination of particular subpopulations with an elevated risk for the occurrence of particular tumours. In a third embodiment of the present invention the frequency may be related to particular stages in tumourigenesis of particular tumours. This embodiment may be e.g. especially useful for the treatment of diagnostically defined tumours or for adjuvant treatment of tumours simultaneously or following primary treatment of tumours.

Using frameshift polypeptides, that occur with a high frequency, the probability, that a particular tumour expresses the peptide and may thus be recognized by antibodies or antigen-recognizing cells, increases. By combination of multiple such polypeptides, the probability to address particular tumour cells further is raised. Preferred frameshift polypeptides according to the present invention occur in at least 25%, more preferred frameshift polypeptides in at least 30% and most preferred frameshift polypeptides in at least 33% of the cases of the particular condition to be treated by the method according to the present invention.

According to the present invention the polypeptides are chosen to be expressed with a high frequency, thus the set of polypeptides may be limited to a number of members and nonetheless may cover a range of occurring diseases as wide as possible. For example a set of 10 polypeptides, each occurring with a frequency of more than 30 percent will statistically cover a range of more than 95% of the potentially existing disorders, as far as they have been included in the studies leading to the respective frequencies. Using 7 polypeptides requires the application of polypeptides with higher frequencies in order to cover a range of about 95% of potentially existing tumours. Using a set of polypeptides derived from enormously frequent mutated microsatellite regions may also allow for the employment of a set of only five different polypeptides without lowering the range of potentially addressed tumours. Thus the set of polypeptides comprises in a preferred embodiment at least 5, in a more preferred embodiment at least 7 and in the most preferred embodiment at least 10 different frameshift polypetides.

A second aspect influencing the choice of a suitable set of frameshift polypeptides concerns the type of mutation found in the microsatellite region. As described above frameshift mutations in microsatellites are usually due to DNA polymerase slippage and may be characterized by the type of the repeat. According to the present invention the polypeptides included within the set shall be selected as to cover the widest possible range of tumours. Thus a set comprises for example (-1) frameshift polypeptides and additionally (+1) frameshift polypeptides. Using more than one possible novel reading frame of the particular gene broadens the spectrum of target cells and thus may prevent escape of particular cells from being eliminated according to the present invention.

A further aspect influencing the choice of the member polypeptides included in a set of frameshift polypeptides according to the present invention is the involvement of the gene encoded for by the coding sequence containing the respective microsatellite in particular biochemical pathways. The term biochemical pathway is used with a rather broad meaning herein. Biochemical pathways as used within this document shall for example include signal transduction pathways, enzymatic pathways, metabolic pathways, the apoptosis pathway, DNA repair or polymerization pathways, the pathway of meiosis etc.. To broaden the spectrum of tumours to be addressed by the set of frameshift polypeptides, members of different pathways are included in the set. In a preferred embodiment at least 5 different pathways, in a more preferred embodiment at least 4 different pathways and in the most preferred embodiment at least 3 different pathways are represented by the frameshift polypeptides in a set according to the present invention. For example the TGFβRII as a member of a signal transduction pathway may be used in combination with the BAX gene as a member of the apoptosis pathway with additional other polypeptides associated with other pathways.

A final aspect influencing the choice of suitable frameshift polypeptides to be included in the set according to the present invention is the length of the novel (poly)peptide sequence arising from the mutation. The shift of the reading frame leads to novel stop codons. Thus the new peptides are not of the same length as the polypeptides naturally encoded by the particular gene. In most cases the new peptides are shorter or even significantly shorter than the original wild-type polypeptide. Frequently rather oligopeptides arise from the frameshift mutations. The fidelity of the immune system in recognizing "foreign" molecules reaches thus far, to identify even polypeptides, that differ from "own" polypeptides in only one single amino-acid mutation. The fragments, bound by the antigen presenting HLA molecules comprise about 12 amino acid residues. To enhance the fidelity of recognition of new polypeptides more than one single amino-acid difference should be present. A polypeptide comprising 3 consecutive amino acids differing from the wild-type amino acid sequence is reliably recognized as foreign by the immune system. This may be due to the increased probability of new amino acid combinations being present in different fragments produced by the antigen presenting machinery. Thus the frameshift polypeptides according to the present invention contain at least one new amino acid, not present in the wild-type polypeptide, in a more preferred embodiment at least 2 new amino acids and in the most preferred embodiment at least 3 new amino acids.

According to the named parameters a basic set of frameshift polypeptides may be tailored, that is suitable to address a large variety of tumours and minimizes the danger of escape of single tumour cells from the therapy. Thus the probability of survival of tumour cells in an organism following immuno-therapy can be minimized and the rate of recurrence of the cancer can be reduced.

A basic set of frameshift polypeptides includes frameshift polypeptides, that do occur with a high mutation frequency in associated disorders. Additionally the polypeptides within the set are chosen to be involved in different biochemical pathways. The mutation types are chosen, that polypeptides of a minimal length of 3 amino acid residues is expressed from the mutated nucleic acid sequence. Furthermore different mutation types of one single microsatellite may be included in the set if applicable.

Additional to the parameters given above, data concerning the particular disorder in focus are taken into account for the design of particular sets of frameshift peptides according to the present invention. Thus individual mutation frequencies, typical mutation types, relevant biochemical pathways or special immunological characteristics may contribute to the set to be used in particular cases. Furthermore in particular cases based on results of examination of particular samples of individuals vaccines may be tailored as to optimally fit the therapy of the respective disorder. In one embodiment of the present invention individual tumour vaccine compositions may be set up according to molecular profiling of individual tumours

Choosing suitable combinations of the peptides within the mixture of frameshift peptides thus enables for generation of vaccines, that elicit immune response specifically for tumours of particular organs. On the other hand it is possible to design sets of frameshift polypeptides that cover a wide range of degenerative disorders or cancers in individuals. The first possibility may be especially useful for the design of curative treatment, whereas the second variant of sets may be of special interest for the design of preventive vaccines.

Monoclonal antibodies directed against frameshift peptides presented on cellular membranes may according to the present invention also be used as therapeutic compounds in order to diminish or eliminate tumours. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include 90Y, 1231, 1251, 1311, 186Re, 188Re, 211At, and 212Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

Pharmaceutical compositions useful in immuno-therapy according to the present invention may comprise a set of at least 5 to 10 frameshift polypeptides (or variants thereof), and a physiologically acceptable carrier. The vaccines may additionally comprise a non-specific immune-response enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of tumour antigens, either incorporated into a fusion protein or present within a separate polypeptide.

Alternatively, a pharmaceutical composition or vaccine suitable for immunotherapy according to the present invention may contain nucleic acids, that code for one or more frameshift polypeptides according to the present invention. Nucleic acids may for example include single-stranded (sense or antisense) or double-stranded molecules, and may be DNA (genomic, cDNA or synthetic) or RNA. RNA molecules comprise as well HnRNA (containing introns) as mRNA (not containing introns). According to the present invention the polynucleotides may also be linked to any other molecules, such as support materials or detection marker molecules, and may, but need not, contain additional coding or non-coding sequences. The nucleic acid may be administered in a way that allows the polypeptides to be generated *in situ.* Suitable expression systems are known to those skilled in the art. The expression of the polypetides may for example be persistent or transient. In pharmaceutical compositions and/or vaccines, providing for *in situ* expression of polypeptides, the nucleic acids may be present within any suitable delivery system known to those of ordinary skill in the art, including nucleic acid expression systems, bacteria and viral expression systems. Appropriate nucleic acid expression systems comprise the necessary regulatory nucleic acid sequences for expression in the patient, such as suitable promoters, terminators etc.. Bacterial delivery systems may for example employ the administration of a bacterium that expresses an epitope of a cell antigen on its cell surface. In a preferred embodiment, the nucleic acid may be introduced using a viral expression system such as e.g., vaccinia virus, retrovirus, or adenovirus, which may involve the use of a non-pathogenic, replication competent virus. Suitable systems are known to those of ordinary skill in the art and are disclosed, for example, in Fisher-Hoch et al., PNAS 86:317-321, 1989; Flexner et al., Ann. N.Y. Acad Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993.

In another embodiment transgenic mammalian cells may be used for delivery and/or expression of the nucleic acids. The methods for producing nucleic acid constructs suitable for *in situ* expression of polypeptides are known to those of skill in the art.

Furthermore the nucleic acid may be administered as naked nucleic acids. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

The pharmaceutical compositions used for immuno-therapy according to the present invention may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according to the parameters, such as age, sex, body weight etc. of the patient, known to those of skill in the art.

The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109. Any of a variety of immune-response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

The pharmaceutical compositions or immuno-therapeutic methods according to the present invention may be used for the treatment of degenerative disorders or cancers. For example the compositions and methods may be employed in the therapy of diagnosed cancers in order to eliminate the tumour cells from the affected organism. As well primary tumours as metastases or disseminated tumour cells within an organism may be targets for the therapeutic compounds and methods disclosed herein.

Furthermore the compositions and methods of the invention may be employed in the treatment of pre-neoplastic conditions. In this case the pre-neoplastic cells or tissues may be directly addressed by the immuno-therapeutic compositions or methods, or may be hindered from evolving into neoplastic or dysplastic conditions. For example in this case the pre-neoplastic condition may be treated preventively. By the vaccination the immune response may be elicited, so that emerging neoplasms may be destroyed.

The methods and compositions according to the present invention may also be used for the prevention of degenerative disorders or cancers associated with frameshift mutations in coding microsatellites. For this purpose a vaccination of a population of organisms or of subgroups of said population may be performed. The subgroups may be built by suitable parameters such as hereditary predisposition for the emergence of degenerative disorders, exposure to factors, that increase the risk of being affected by said disorders etc.

The present invention provides compositions and methods for enhanced diagnosis and therapy of disorders associated with MSI⁺ related occurrence of frameshift peptides. The invention provides new frameshift polypeptides for diagnosis therapy of said disorders, that may be used each alone or in combinations tailored, to address a wide range of different types of disorders in an organism and may thus be employed as a preventive vaccine against said disorders.

### Brief description of the drawings:

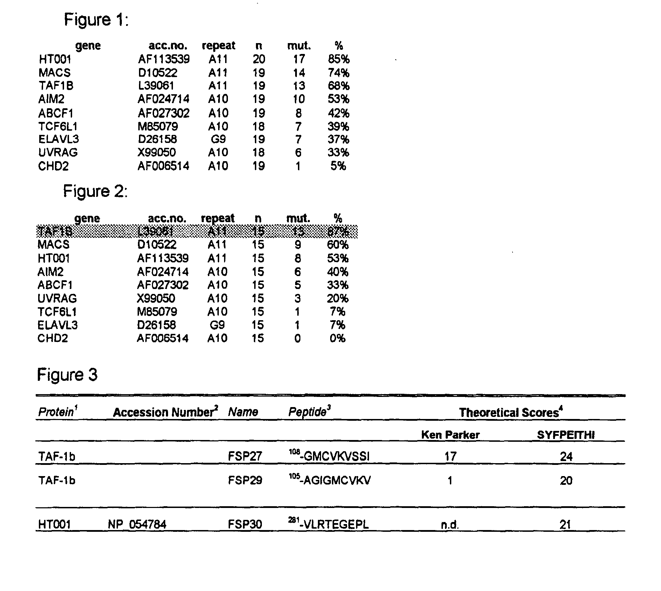

- Figure 1:: **Coding microsatellite genes investigated in MSI-H colorectal cancer.** Repeat: type of nucleotide and length of repeat tract, n: number of samples investigated, mut.: number of samples mutated, %: percentage of mutated samples,
- Figure 2:: **Coding microsatellite genes investigated in MSI-H gastric cancer.** Repeat: type of nucleotide and length of repeat tract, n: number of samples investigated, mut.: number of samples mutated, %: percentage of mutated samples
- Figure 3:: **Frameshift Peptides analysed for in vitro stimulation of a cellular immune response;** All analysed peptides are derived from (-1) mutations in microsatellites of the cognate proteins; The protein or nucleotide accession numbers are indicated within the table; the position of the start amino acid in the protein is indicated in the tables; the predicted binding scores to HLA-A2.1 using computer assisted analysis; for experimental details see Example 3.

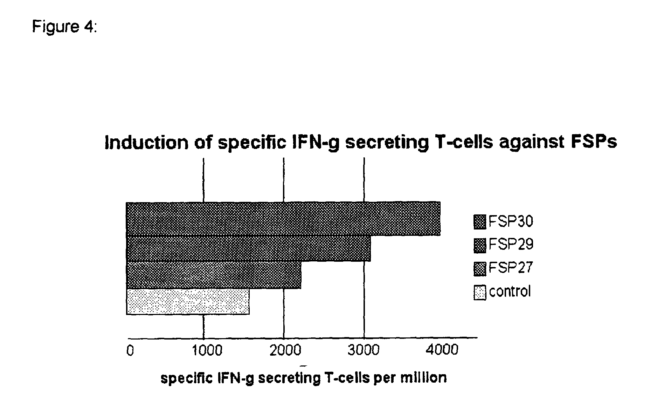

- Figure 4:: **ELISpot-analysis of FSP T-cell lines** (FS27, FSP29 and FSP30). Titrated amounts of T-cells were incubated overnight with 3.5x10⁴ peptide loaded T2 cells per well. The number of IFN-γ-releasing activated T-cells (spots) among the total number of cells analyzed (10⁶) is depicted for each frameshift peptide. Reactivity against peptide YLLPAIVHI from the nuclear protein P68 served as a negative control and is indicated (control).

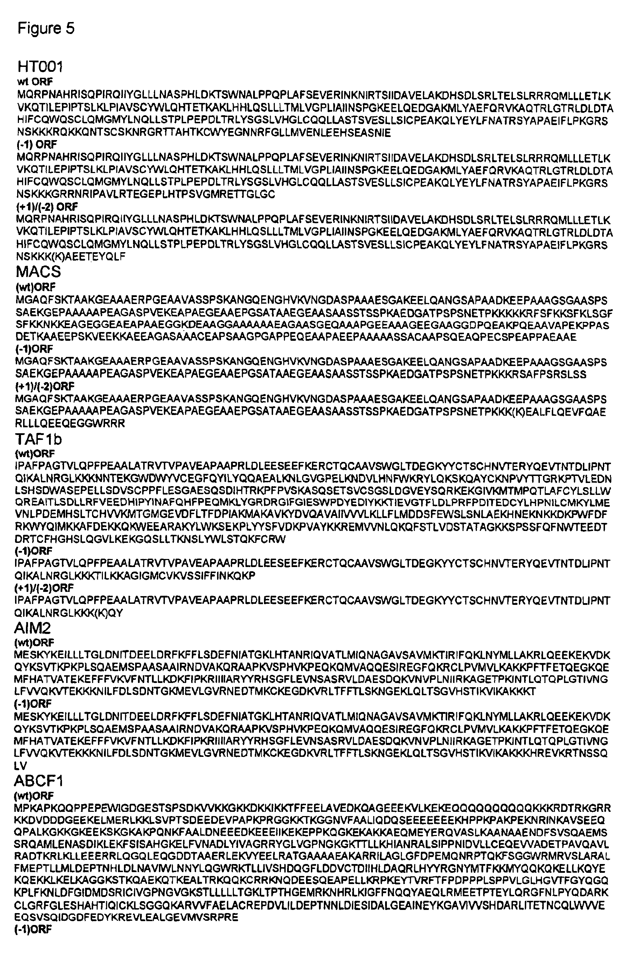

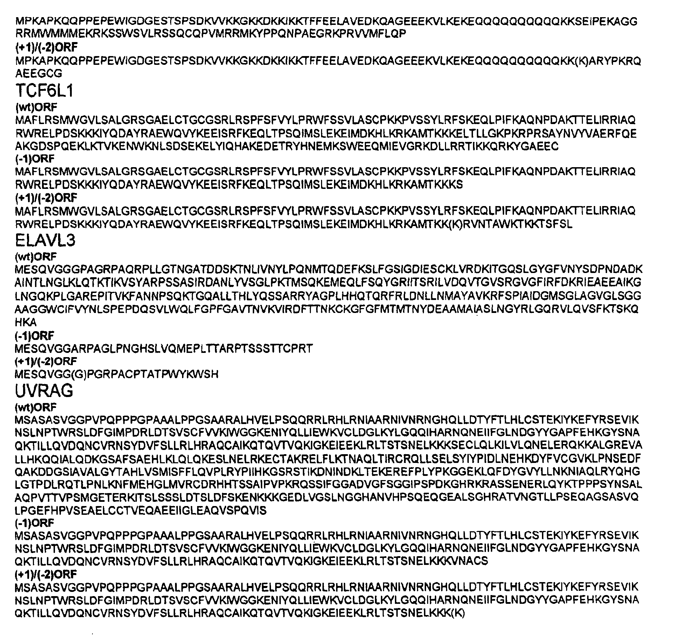

- Figure 5:: **Listing of sequences of polypeptides encoded by genes with coding microsatellites;** the sequences of polypeptides arising from different possible frameshift mutations are depicted. For each polypeptide the sequence expressed from the wild type open reading frames is given (wtORF); Furthermore the sequences expressed from (-1) mutations and from (-2/+1) mutation are given.

The following examples are given for illustration of the invention only and are not intended to limit the scope of the invention.

### Example 1: Analysis of the mutation frequency of genes harbouring repeat tracts

Investigations were performed regarding the mutation frequencies of unpublished coding microsatellite regions. Nine novel coding microsatellites residing in genes not yet analyzed for frameshift mutations in MSI colorectal, endometrial or gastric tumours have been examined in the course of the studies leading to the present invention. They include three genes containing A11 repeats (TAF1B, MACS, HT001), five genes with A10 repeats (CHD2, UVRAG, TCF6L1, ABCF1, AIM2) and one gene harboring a G9 repeat (ELAVL3). The MSI status of these specimens was determined using the NCI ICG-HNPCC microsatellite reference marker panel (8). PCR reactions were performed as follows:

Genomic DNA was isolated from 5-8 haematoxylin and eosin stained 5 µm sections after microdissection, using the Qiamp Tissue Kit (Qiagen, Hilden, Germany). Preparation of DNA from cell lines was performed according to standard protocols. PCR primers were designed to closely flank the target sequence, yielding short amplimeres of about 100 base pairs thus allowing precise fragment sizing and robust amplification from archival tissues (Table 1). PCR reactions were performed in a total volume of 25 µl containing 50 ng genomic DNA, 2.5 µl 10 x reaction buffer (Life Technologies, Karlsruhe, Germany), 1.5 mM MgCl₂, 200 µM dNTPs, 0.3 µM of each primer, and 0.5 U Taq DNA polymerase (Life Technologies) and using the following conditions: initial denaturation at 94 °C for 4 min, followed by 35 cycles of denaturation at 94 °C for 30 s, annealing at 58 °C for 45 s, and primer extension at 72 °C for 30 s. The final extension step was carried out at 72 °C for 6 min. PCR fragments were analyzed on an ALF DNA sequencing device (Amersham Pharmacia Biotech, Freiburg, Germany) using 6.6 % polyacrylamide/ 7 M urea gels. Size, height and profile of microsatellite peaks were analyzed using the AlleleLinks software (Amersham Pharmacia Biotech). Coding microsatellite instability was scored, if smaller or larger-sized amplimeres were detected in tumour DNA compared to DNA from non-neoplastic cells. Allele intensities were determined and ratios of wild-type and novel alleles in normal and tumour tissues were calculated, defining a two-fold difference as threshold for allelic shifts. Similarly, unstable alleles in tumour cell lines were identified by comparison to 36 unmatched normal mucosae. In order to determine the predicted repeat type and length amplified coding microsatellites were subjected to Big Dye terminator cycle sequencing (Perkin Elmer, Darmstadt, Germany) and subsequent analysis on an ABI 310 sequencing device.

The frequencies of mutations in a particular microsatellite region examined herein are given in figure 1/2. The mutation rates are calculated with respect to the total number of samples included in the study.

The results show, that the tested microsatellite regions are frequently mutated in tumour samples.

### Example 2: Detection of the expression of frameshift mutated mRNA from genes harboring coding microsatellite regions using PCR

Samples of colon, gastric and endometrial carcinomas are used to determine the expression of mRNA showing frameshift mutations in coding microsatellite regions using PCR and subsequent sequencing of the amplified nucleic acid products.

Tumours are collected, snap frozen, and stored at -80°C. They are verified to be composed predominantly of neoplastic cells by histopathological analysis. mRNA is isolated from tumours using Qiagen reagents (Qiagen, Hilden, Germany), and single-stranded cDNA is synthesized using Superscript II (Life Technologies, Inc.).

PCR reactions were performed in a total volume of 25 µl containing 50 ng cDNA, 2.5 µl 10 x reaction buffer (Life Technologies, Karlsruhe, Germany), 1.5 mM MgCl₂, 200 µM dNTPs, 0.3 µM of each primer, and 0.5 U Taq DNA polymerase (Life Technologies) and using the following conditions: initial denaturation at 94 °C for 4 min, followed by 35 cycles of denaturation at 94 °C for 30 s, annealing at 58 °C for 45 s, and primer extension at 72 °C for 30 s. The final extension step was carried out at 72 °C for 6 min. PCR fragments were analyzed on an ALF DNA sequencing device (Amersham Pharmacia Biotech, Freiburg, Germany) using 6.6 % polyacrylamide/ 7 M urea gels.

The experiments described show, that in the cases of the tested genes CHD2, UVRAG, ELAVL3, TCF6L1, ABCF1, AIM2, TAF1B, MACS and HT001 mutated coding microsatellite regions are transcribed into mRNA.

These results indicate, that the cells harbouring mutations in coding microsatellite regions of the nine genes tested express neo-polypeptides derived from these frameshift mutations.

### Example 3: Stimulation of cellular immune response by frameshift peptides

The present experiments were performed in order to determine, whether the frameshift peptides arising from mutations in coding microsatellite regions according to the present invention are suited to stimulate a cellular immune response. The experiments were performed as follows:

Peptides displaying HLA-A2.1-binding motifs were selected by taking advantage of specific computer programs [(Parker, Bednarek, & Coligan 1994); http://bimas.dcrt.nih.gov/molbio/hla_bind/ and (Rammensee et al. 1999); http://134.2.96.221/scripts/MHCServer.dll/home.htm]. Peptides were purchased from the peptide synthesis unit of the DKFZ. Stock solutions (10mg/ml in DMSO) were stored at -70°C and diluted to 1mg/ml in PBS before use. T2 cells were pulsed with 50µg/ml peptide and 5µg/ml β2-microglobulin (Sigma; Deisenhofen, Germany) overnight at 37°C. The expression of HLA-A2.1 was then analysed by flow cytometry using mAb BB7.2 followed by incubation with FITC-conjugated (ab')2 goat anti-mouse Ig (Vonderheide et al. 1999).

Peripheral blood was obtained from a healthy HLA-A2.1+ donor and collected in heparinized tubes. PBMNC were isolated by Ficoll-density gradient centrifugation. Whole CD3+ T-cells were isolated from PBMNC by magnetic depletion of non-T-cells using the MACS Pan T-cell Isolation Kit (Miltenyi; Bergisch Gladbach, Germany) according to manufacturer's instructions. Preparations contained at least 97% of CD3+ cells as assessed by immunophenotypic analysis.

HLA-A2.1-restricted peptides were FSP27 and FSP29 from a (-1) in the TAF1B-gene; FSP30 was derived from a (-1) mutation in the HT001 gene.

CD40 Bs of a HLA-A2.1+ donor were incubated with peptide (10µg/ml) and human β2-microglobulin (3µg/ml; Sigma) in serum-free Iscov's DMEM medium for one hour at room temperature, washed twice to remove excess of peptide, were irradiated (30Gy) and added to purified CD3+ autologous T-cells (>97% CD3+) at a ratio of 4:1 (T:CD40 Bs) in Iscov's MEM containing 10% human AB-serum, supplements (1:100) and hlL-7 (10ng/ml, R&D). Cells were plated at a density of 2 x 106 T-cells/well in 1ml of medium. After three days in culture they were fed with 1ml complete medium. For restimulation of T-cells, this was repeated weekly. IL-2 was first given at day 21 (10IU/ml, R&D), also at day 24 and from day 28 on only hlL-2 was used instead of hlL7.

ELISpot assays were performed as described elsewhere (Meyer et al. 1998). Briefly, nitrocellulose-96-well plates (Multiscreen; Millipore, Bedford, USA) were covered with mouse anti-human IFN-g monoclonal antibody (Mabtech, Sweden) and blocked with serum containing medium. Varying numbers of effector cells were plated in triplicates with 3,5x104 peptide-loaded T2 cells per well as targets. After incubation for 18h, plates were washed, incubated with biotinylated rabbit anti-human IFN-g second antibody, washed again, incubated with streptavidin coupled alkaline phosphatase, followed by a final wash. Spots were detected by incubation with NBT/BCIP (Sigma) for 45min, reaction was stopped with water, after drying spots were counted using the KS-ELISpot reader (Zeiss Kontron; Göttingen, Germany).

The analysis shows, that the used peptides are suited to raise an immune response. Peptides arising from frameshift mutations thus may be used to raise immune response for example in the course of vaccinations according to the present invention.

### Example 4: Screening for Antibodies directed against frameshift peptides in patient samples

Sera of 25 patients with diagnosed colorectal carcinomas were tested for the presence of antibodies to a set of frameshift peptides arising from frameshift mutations in coding microsatellite regions of the following genes: CHD2, UVRAG, ELAVL3, TCF6L1, ABCF1, AIM2, TAF1B, MACS and HT001. As a control the sera of 50 normal individuals were tested.

For the test synthetic peptides representing immunogenic portions of all relevant frameshift peptides (see figure 5) arising from the respective genes were spotted onto nylon membranes. The nylon membranes were subsequently incubated for one hour in phosphate-buffered saline (PBS) with 5% milk powder for blocking unspecific membrane binding. After washing the membranes 3x with PBS, the membranes were incubated with the test and control sera. The sera were diluted 1:1.000 in PBS/0,5% milk powder and incubated overnight with gentle shaking. Subsequently the sera were removed, and membranes were washed three times in PBS before they were incubated with a polyclonal alkaline phosphatase conjugated goat anit-human IgG antibody for one hour. Thereafter, the membranes were washed repeatedly with PBS/0,05% TWEEN20 before staining reaction was developed using nitroblue tetrazolium chloride and bromochoro-indoyl-phosphate (SigmaAldrich) in Tris-buffered saline (TBS). Binding of human antibodies specific for individual frameshift polypeptides thus was made visible by color-deposit on the respective membrane.

The results show, that in all samples of tumour patients antibodies directed against at least one peptide arising from frameshift mutations were present.

This illustrates, that the method according to the present invention may be used for diagnosis of diseases associated with frameshift mutations in coding regions of genes.

## Claims

1. A nucleic acid sequence encoding a polypeptide selected from a group consisting of CHD2, UVRAG, ELAVL3, TCF6L1, ABCF1, AIM2, TAF1B, MACS and HT001 having a frameshift mutation and being associated with gastrointestinal cancer, wherein the frameshift mutation is selected from a group comprising the following:
a. the insertion of one A in the A11 repeats of the genes TAF1B, MACS, HT001
b. the insertion of two A in the A11 repeats of the genes TAF1B, MACS, HT001
c. the deletion of one A in the A11 repeats of the genes TAF1B, MACS, HT001
d. the deletion of two A in the A11 repeats of the genes TAF1B, MACS, HT001
e. the insertion of one A in the A10 repeats of the genes CHD2, UVRAG, TCF6L1, ABCF1, AIM2
f. the insertion of two A in the A10 repeats of the genes CHD2, UVRAG, TCF6L1, ABCF1, AIM2
g. the deletion of one A in the A10 repeats of the genes CHD2, UVRAG, TCF6L1, ABCF1, AIM2
h. the deletion of two A in the A10 repeats of the genes CHD2, UVRAG, TCF6L1, ABCF1, AIM2
i. the insertion of one G in the G9 repeat of the gene ELAVL3
j. the insertion of two G in the G9 repeat of the gene ELAVL3
k. the deletion of one G in the G9 repeat of the gene ELAVL3
l. the deletion of two G in the G9 repeat of the gene ELAVL3

2. The nucleic acid according to claim 1 for use in detection of disorders associated with frameshift mutations in coding microsatellite regions of the CHD2, UVRAG, ELAVL3, TCF6L1, ABCF1, AIM2, TAF1B, MACS and/or HT001 genes and/or for use in preparation of pharmaceutical compositions for treatment of disorders associated with frameshift mutations in said coding microsatellite regions.

3. A frameshift polypeptide associated with gastrointestinal cancers selected from a group comprising:
a. a frameshift polypeptide derived from a frameshift mutation as described in claim 1 in a coding microsatellite region of a gene selected from the group consisting of CHD2, UVRAG, ELAVL3, TCF6L1, ABCF1, AIM2, TAF1B, MACS and HT001;
b. a polypeptide described by an amino acid sequence given in Figure 5 ;
c. a polypeptide encoded by a nucleic acid sequence according to claim 1;
d. a fragment or a portion of the polypeptides of a) to c) containing at least one amino acid not present in the wild-type protein.

4. A frameshift polypeptide according to claim 3 for use in detection of disorders associated with frameshift mutations in coding microsatellite regions of the CHD2, UVRAG, ELAVL3, TCF6L1, ABCF1, AIM2, TAF1B, MACS and/or HT001 genes and/or for use in preparation of pharmaceutical compositions for treatment of disorders associated with frameshift mutations in said coding microsatellite regions.

5. A method for treatment of disorders associated with frameshift mutations in coding microsatellites of the CHD2, UVRAG, ELAVL3, TCF6L1, ABCF1, AIM2, TAF1B, MACS and/or HT001 genes comprising administering one or more frameshift polypeptides according to claim 3 or nucleic acid sequences according to claim 1 in a pharmaceutical acceptable form to an individual.

6. The method of claim 5 additionally comprising administering one or more further frameshift polypeptides arising from frameshift mutations in coding microsatellite regions.

7. The methods according to the claims 5 or 6, wherein the disorder is a degenerative disorder.

8. The methods according to the claims 5 or 6, wherein the disorder is a neurodegnerative disorder, vascular disease, cancer or precursory stages of cancer.

9. The methods according to any one of the claims 5-8, wherein the treatment is immuno-therapeutic treatment of disorders.

10. The methods according to any one of the claims 5-8, wherein the treatment is preventive vaccination against disorders.

11. A pharmaceutical composition for use in the methods according to any one of the claims 5-10 comprising a nucleic acid according to claim 1 and/or a polypeptide according to claim 3 in physiological acceptable form.

12. A method for detection of a disorder associated with frameshift mutations in coding microsatellite regions comprising detecting the presence or absence of one or more nucleic acids according to claim 1 and/or frameshift polypeptides according to claim 3 in a biological sample.

13. A method for detection of a disorder associated with frameshift mutations in coding microsatellite regions comprising detecting the presence or absence of antibodies directed against one or more frameshift polypeptides according to claim 3 in a biological sample.

14. A method for detection of a disorder associated with frameshift mutations in coding microsatellite regions comprising detecting the presence or absence of cells specifically directed against one or more frameshift polypeptides according to claim 3 in a biological sample.

15. The methods according to any one of the claims 12-14, wherein the disorder is a degenerative disorder

16. The methods according to any one of the claims 12-14, wherein the disorder is a neurodegenerative disorder, vascular disease, cancer or precursory stages of cancer.

17. A diagnostic or research kit for performing the methods according to any one of the claims 12-16 comprising one or more nucleic acids according to claim 1 and/or one or more polypeptides according to claim 3.


SEQUENCE LISTING

<110> MTM Laboratories AG

<120> Compounds useful for dectection and treatment of cancer

<130> M4493

<140> EP 02 008 771.4
<141> 2002-04-18

<160> 23

<170> PatentIn Ver. 2.1

<210> 1
<211> 320
<212> PRT
<213> Homo sapiens

<400> 1
Met Gln Arg Pro Asn Ala His Arg Ile Ser Gln Pro Ile Arg Gln Ile
1               5                   10                  15

Ile Tyr Gly Leu Leu Leu Asn Ala Ser Pro His Leu Asp Lys Thr Ser
            20                  25                  30

Trp Asn Ala Leu Pro Pro Gln Pro Leu Ala Phe Ser Glu Val Glu Arg
        35                  40                  45

Ile Asn Lys Asn Ile Arg Thr Ser Ile Ile Asp Ala Val Glu Leu Ala
    50                  55                  60

Lys Asp His Ser Asp Leu Ser Arg Leu Thr Glu Leu Ser Leu Arg Arg
65                  70                  75                  80

Arg Gln Met Leu Leu Leu Glu Thr Leu Lys Val Lys Gln Thr Ile Leu
                85                  90                  95

Glu Pro Ile Pro Thr Ser Leu Lys Leu Pro Ile Ala Val Ser Cys Tyr
            100                 105                 110

Trp Leu Gln His Thr Glu Thr Lys Ala Lys Leu His His Leu Gln Ser
        115                 120                 125

Leu Leu Leu Thr Met Leu Val Gly Pro Leu Ile Ala Ile Ile Asn Ser
    130                 135                 140

Pro Gly Lys Glu Glu Leu Gln Glu Asp Gly Ala Lys Met Leu Tyr Ala
145                 150                 155                 160

Glu Phe Gln Arg Val Lys Ala Gln Thr Arg Leu Gly Thr Arg Leu Asp
                165                 170                 175

Leu Asp Thr Ala His Ile Phe Cys Gln Trp Gln Ser Cys Leu Gln Met
            180                 185                 190

Gly Met Tyr Leu Asn Gln Leu Leu Ser Thr Pro Leu Pro Glu Pro Asp
        195                 200                 205

Leu Thr Arg Leu Tyr Ser Gly Ser Leu Val His Gly Leu Cys Gln Gln
    210                 215                 220

Leu Leu Ala Ser Thr Ser Val Glu Ser Leu Leu Ser Ile Cys Pro Glu
225                 230                 235                 240

Ala Lys Gln Leu Tyr Glu Tyr Leu Phe Asn Ala Thr Arg Ser Tyr Ala
                245                 250                 255

Pro Ala Glu Ile Phe Leu Pro Lys Gly Arg Ser Asn Ser Lys Lys Lys
            260                 265                 270

Arg Gln Lys Lys Gln Asn Thr Ser Cys Ser Lys Asn Arg Gly Arg Thr
        275                 280                 285

Thr Ala His Thr Lys Cys Trp Tyr Glu Gly Asn Asn Arg Phe Gly Leu
    290                 295                 300

Leu Met Val Glu Asn Leu Glu Glu His Ser Glu Ala Ser Asn Ile Glu
305                 310                 315                 320

<210> 2
<211> 304
<212> PRT
<213> Homo sapiens

<400> 2
Met Gln Arg Pro Asn Ala His Arg Ile Ser Gln Pro Ile Arg Gln Ile
  1               5                  10                  15

Ile Tyr Gly Leu Leu Leu Asn Ala Ser Pro His Leu Asp Lys Thr Ser
             20                  25                  30

Trp Asn Ala Leu Pro Pro Gln Pro Leu Ala Phe Ser Glu Val Glu Arg
         35                  40                  45

Ile Asn Lys Asn Ile Arg Thr Ser Ile Ile Asp Ala Val Glu Leu Ala
     50                  55                  60

Lys Asp His Ser Asp Leu Ser Arg Leu Thr Glu Leu Ser Leu Arg Arg
 65                  70                  75                  80

Arg Gln Met Leu Leu Leu Glu Thr Leu Lys Val Lys Gln Thr Ile Leu
                 85                  90                  95

Glu Pro Ile Pro Thr Ser Leu Lys Leu Pro Ile Ala Val Ser Cys Tyr
            100                 105                 110

Trp Leu Gln His Thr Glu Thr Lys Ala Lys Leu His His Leu Gln Ser
        115                 120                 125

Leu Leu Leu Thr Met Leu Val Gly Pro Leu Ile Ala Ile Ile Asn Ser
    130                 135                 140

Pro Gly Lys Glu Glu Leu Gln Glu Asp Gly Ala Lys Met Leu Tyr Ala
145                 150                 155                 160

Glu Phe Gln Arg Val Lys Ala Gln Thr Arg Leu Gly Thr Arg Leu Asp
                165                 170                 175

Leu Asp Thr Ala His Ile Phe Cys Gln Trp Gln Ser Cys Leu Gln Met
            180                 185                 190

Gly Met Tyr Leu Asn Gln Leu Leu Ser Thr Pro Leu Pro Glu Pro Asp
        195                 200                 205

Leu Thr Arg Leu Tyr Ser Gly Ser Leu Val His Gly Leu Cys Gln Gln
    210                 215                 220

Leu Leu Ala Ser Thr Ser Val Glu Ser Leu Leu Ser Ile Cys Pro Glu
225                 230                 235                 240

Ala Lys Gln Leu Tyr Glu Tyr Leu Phe Asn Ala Thr Arg Ser Tyr Ala
                245                 250                 255

Pro Ala Glu Ile Phe Leu Pro Lys Gly Arg Ser Asn Ser Lys Lys Lys
            260                 265                 270

Gly Arg Arg Asn Arg Ile Pro Ala Val Leu Arg Thr Glu Gly Glu Pro
        275                 280                 285

Leu His Thr Pro Ser Val Gly Met Arg Glu Thr Thr Gly Leu Gly Cys
    290                 295                 300

<210> 3
<211> 282
<212> PRT
<213> Homo sapiens

<400> 3
Met Gln Arg Pro Asn Ala His Arg Ile Ser Gln Pro Ile Arg Gln Ile
  1               5                  10                  15

Ile Tyr Gly Leu Leu Leu Asn Ala Ser Pro His Leu Asp Lys Thr Ser
             20                  25                  30

Trp Asn Ala Leu Pro Pro Gln Pro Leu Ala Phe Ser Glu Val Glu Arg
         35                  40                  45

Ile Asn Lys Asn Ile Arg Thr Ser Ile Ile Asp Ala Val Glu Leu Ala
     50                  55                  60

Lys Asp His Ser Asp Leu Ser Arg Leu Thr Glu Leu Ser Leu Arg Arg
 65                  70                  75                  80

Arg Gln Met Leu Leu Leu Glu Thr Leu Lys Val Lys Gln Thr Ile Leu
                 85                  90                  95

Glu Pro Ile Pro Thr Ser Leu Lys Leu Pro Ile Ala Val Ser Cys Tyr
            100                 105                 110

Trp Leu Gln His Thr Glu Thr Lys Ala Lys Leu His His Leu Gln Ser
        115                 120                 125

Leu Leu Leu Thr Met Leu Val Gly Pro Leu Ile Ala Ile Ile Asn Ser
    130                 135                 140

Pro Gly Lys Glu Glu Leu Gln Glu Asp Gly Ala Lys Met Leu Tyr Ala
145                 150                 155                 160

Glu Phe Gln Arg Val Lys Ala Gln Thr Arg Leu Gly Thr Arg Leu Asp
                165                 170                 175

Leu Asp Thr Ala His Ile Phe Cys Gln Trp Gln Ser Cys Leu Gln Met
            180                 185                 190

Gly Met Tyr Leu Asn Gln Leu Leu Ser Thr Pro Leu Pro Glu Pro Asp
        195                 200                 205

Leu Thr Arg Leu Tyr Ser Gly Ser Leu Val His Gly Leu Cys Gln Gln
    210                 215                 220

Leu Leu Ala Ser Thr Ser Val Glu Ser Leu Leu Ser Ile Cys Pro Glu
225                 230                 235                 240

Ala Lys Gln Leu Tyr Glu Tyr Leu Phe Asn Ala Thr Arg Ser Tyr Ala
                245                 250                 255

Pro Ala Glu Ile Phe Leu Pro Lys Gly Arg Ser Asn Ser Lys Lys Lys
            260                 265                 270

Lys Ala Glu Glu Thr Glu Tyr Gln Leu Phe
        275                 280


<210> 4
<211> 332
<212> PRT
<213> Homo sapiens

<400> 4
Met Gly Ala Gln Phe Ser Lys Thr Ala Ala Lys Gly Glu Ala Ala Ala
1               5                   10                  15

Glu Arg Pro Gly Glu Ala Ala Val Ala Ser Ser Pro Ser Lys Ala Asn
            20                  25                  30

Gly Gln Glu Asn Gly His Val Lys Val Asn Gly Asp Ala Ser Pro Ala
        35                  40                  45

Ala Ala Glu Ser Gly Ala Lys Glu Glu Leu Gln Ala Asn Gly Ser Ala
    50                  55                  60

Pro Ala Ala Asp Lys Glu Glu Pro Ala Ala Ala Gly Ser Gly Ala Ala
65                  70                  75                  80

Ser Pro Ser Ser Ala Glu Lys Gly Glu Pro Ala Ala Ala Ala Ala Pro
                85                  90                  95

Glu Ala Gly Ala Ser Pro Val Glu Lys Glu Ala Pro Ala Glu Gly Glu
            100                 105                 110

Ala Ala Glu Pro Gly Ser Ala Thr Ala Ala Glu Gly Glu Ala Ala Ser
        115                 120                 125

Ala Ala Ser Ser Thr Ser Ser Pro Lys Ala Glu Asp Gly Ala Thr Pro
    130                 135                 140

Ser Pro Ser Asn Glu Thr Pro Lys Lys Lys Lys Lys Arg Phe Ser Phe
145                 150                 155                 160

Lys Lys Ser Phe Lys Leu Ser Gly Phe Ser Phe Lys Lys Asn Lys Lys
                165                 170                 175

Glu Ala Gly Glu Gly Gly Glu Ala Glu Ala Pro Ala Ala Glu Gly Gly
            180                 185                 190

Lys Asp Glu Ala Ala Gly Gly Ala Ala Ala Ala Ala Ala Glu Ala Gly
        195                 200                 205

Ala Ala Ser Gly Glu Gln Ala Ala Ala Pro Gly Glu Glu Ala Ala Ala
    210                 215                 220

Gly Glu Glu Gly Ala Ala Gly Gly Asp Pro Gln Glu Ala Lys Pro Gln
225                 230                 235                 240

Glu Ala Ala Val Ala Pro Glu Lys Pro Pro Ala Ser Asp Glu Thr Lys
                245                 250                 255

Ala Ala Glu Glu Pro Ser Lys Val Glu Glu Lys Lys Ala Glu Glu Ala
            260                 265                 270

Gly Ala Ser Ala Ala Ala Cys Glu Ala Pro Ser Ala Ala Gly Pro Gly
        275                 280                 285

Ala Pro Pro Glu Gln Glu Ala Ala Pro Ala Glu Glu Pro Ala Ala Ala
    290                 295                 300

Ala Ala Ser Ser Ala Cys Ala Ala Pro Ser Gln Glu Ala Gln Pro Glu
305                 310                 315                 320

Cys Ser Pro Glu Ala Pro Pro Ala Glu Ala Ala Glu
                325                 330


<210> 5
<211> 165
<212> PRT
<213> Homo sapiens

<400> 5
Met Gly Ala Gln Phe Ser Lys Thr Ala Ala Lys Gly Glu Ala Ala Ala
  1               5                  10                  15

Glu Arg Pro Gly Glu Ala Ala Val Ala Ser Ser Pro Ser Lys Ala Asn
             20                  25                  30

Gly Gln Glu Asn Gly His Val Lys Val Asn Gly Asp Ala Ser Pro Ala
         35                  40                  45

Ala Ala Glu Ser Gly Ala Lys Glu Glu Leu Gln Ala Asn Gly Ser Ala
     50                  55                  60

Pro Ala Ala Asp Lys Glu Glu Pro Ala Ala Ala Gly Ser Gly Ala Ala
 65                  70                  75                  80

Ser Pro Ser Ser Ala Glu Lys Gly Glu Pro Ala Ala Ala Ala Ala Pro
                 85                  90                  95

Glu Ala Gly Ala Ser Pro Val Glu Lys Glu Ala Pro Ala Glu Gly Glu
            100                 105                 110

Ala Ala Glu Pro Gly Ser Ala Thr Ala Ala Glu Gly Glu Ala Ala Ser
        115                 120                 125

Ala Ala Ser Ser Thr Ser Ser Pro Lys Ala Glu Asp Gly Ala Thr Pro
    130                 135                 140

Ser Pro Ser Asn Glu Thr Pro Lys Lys Lys Arg Ser Ala Phe Pro Ser
145                 150                 155                 160

Arg Ser Leu Ser Ser
                165


<210> 6
<211> 182
<212> PRT
<213> Homo sapiens

<400> 6
Met Gly Ala Gln Phe Ser Lys Thr Ala Ala Lys Gly Glu Ala Ala Ala
  1               5                  10                  15

Glu Arg Pro Gly Glu Ala Ala Val Ala Ser Ser Pro Ser Lys Ala Asn
             20                  25                  30

Gly Gln Glu Asn Gly His Val Lys Val Asn Gly Asp Ala Ser Pro Ala
            35                  40                  45

Ala Ala Glu Ser Gly Ala Lys Glu Glu Leu Gln Ala Asn Gly Ser Ala
        50                  55                  60

Pro Ala Ala Asp Lys Glu Glu Pro Ala Ala Ala Gly Ser Gly Ala Ala
65                  70                  75                  80

Ser Pro Ser Ser Ala Glu Lys Gly Glu Pro Ala Ala Ala Ala Ala Pro
                85                  90                  95

Glu Ala Gly Ala Ser Pro Val Glu Lys Glu Ala Pro Ala Glu Gly Glu
            100                 105                 110

Ala Ala Glu Pro Gly Ser Ala Thr Ala Ala Glu Gly Glu Ala Ala Ser
        115                 120                 125

Ala Ala Ser Ser Thr Ser Ser Pro Lys Ala Glu Asp Gly Ala Thr Pro
    130                 135                 140

Ser Pro Ser Asn Glu Thr Pro Lys Lys Lys Lys Glu Ala Leu Phe Leu
145                 150                 155                 160

Gln Glu Val Phe Gln Ala Glu Arg Leu Leu Leu Gln Glu Glu Gln Glu
                165                 170                 175

Gly Gly Trp Arg Arg Arg
            180


<210> 7
<211> 556
<212> PRT
<213> Homo sapiens

<400> 7
Ile Pro Ala Phe Pro Ala Gly Thr Val Leu Gln Pro Phe Pro Glu Ala
1               5                   10                  15

Ala Leu Ala Thr Arg Val Thr Val Pro Ala Val Glu Ala Pro Ala Ala
            20                  25                  30

Pro Arg Leu Asp Leu Glu Glu Ser Glu Glu Phe Lys Glu Arg Cys Thr
        35                  40                  45

Gln Cys Ala Ala Val Ser Trp Gly Leu Thr Asp Glu Gly Lys Tyr Tyr
    50                  55                  60

Cys Thr Ser Cys His Asn Val Thr Glu Arg Tyr Gln Glu Val Thr Asn
65                  70                  75                  80

Thr Asp Leu Ile Pro Asn Thr Gln Ile Lys Ala Leu Asn Arg Gly Leu
                85                  90                  95

Lys Lys Lys Asn Asn Thr Glu Lys Gly Trp Asp Trp Tyr Val Cys Glu
            100                 105                 110

Gly Phe Gln Tyr Ile Leu Tyr Gln Gln Ala Glu Ala Leu Lys Asn Leu
        115                 120                 125

Gly Val Gly Pro Glu Leu Lys Asn Asp Val Leu His Asn Phe Trp Lys
    130                 135                 140

Arg Tyr Leu Gln Lys Ser Lys Gln Ala Tyr Cys Lys Asn Pro Val Tyr
145                 150                 155                 160

Thr Thr Gly Arg Lys Pro Thr Val Leu Glu Asp Asn Leu Ser His Ser
                165                 170                 175

Asp Trp Ala Ser Glu Pro Glu Leu Leu Ser Asp Val Ser Cys Pro Pro
            180                 185                 190

Phe Leu Glu Ser Gly Ala Glu Ser Gln Ser Asp Ile His Thr Arg Lys
        195                 200                 205

Pro Phe Pro Val Ser Lys Ala Ser Gln Ser Glu Thr Ser Val Cys Ser
    210                 215                 220

Gly Ser Leu Asp Gly Val Glu Tyr Ser Gln Arg Lys Glu Lys Gly Ile
225                 230                 235                 240

Val Lys Met Thr Met Pro Gln Thr Leu Ala Phe Cys Tyr Leu Ser Leu
                245                 250                 255

Leu Trp Gln Arg Glu Ala Ile Thr Leu Ser Asp Leu Leu Arg Phe Val
            260                 265                 270

Glu Glu Asp His Ile Pro Tyr Ile Asn Ala Phe Gln His Phe Pro Glu
        275                 280                 285

Gln Met Lys Leu Tyr Gly Arg Asp Arg Gly Ile Phe Gly Ile Glu Ser
    290                 295                 300

Trp Pro Asp Tyr Glu Asp Ile Tyr Lys Lys Thr Ile Glu Val Gly Thr
305                 310                 315                 320

Phe Leu Asp Leu Pro Arg Phe Pro Asp Ile Thr Glu Asp Cys Tyr Leu
                325                 330                 335

His Pro Asn Ile Leu Cys Met Lys Tyr Leu Met Glu Val Asn Leu Pro
            340                 345                 350

Asp Glu Met His Ser Leu Thr Cys His Val Val Lys Met Thr Gly Met
        355                 360                 365

Gly Glu Val Asp Phe Leu Thr Phe Asp Pro Ile Ala Lys Met Ala Lys
    370                 375                 380

Ala Val Lys Tyr Asp Val Gln Ala Val Ala Ile Ile Val Val Val Leu
385                 390                 395                 400

Lys Leu Leu Phe Leu Met Asp Asp Ser Phe Glu Trp Ser Leu Ser Asn
                405                 410                 415

Leu Ala Glu Lys His Asn Glu Lys Asn Lys Lys Asp Lys Pro Trp Phe
            420                 425                 430

Asp Phe Arg Lys Trp Tyr Gln Ile Met Lys Lys Ala Phe Asp Glu Lys
        435                 440                 445

Lys Gln Lys Trp Glu Glu Ala Arg Ala Lys Tyr Leu Trp Lys Ser Glu
    450                 455                 460

Lys Pro Leu Tyr Tyr Ser Phe Val Asp Lys Pro Val Ala Tyr Lys Lys
465                 470                 475                 480

Arg Glu Met Val Val Asn Leu Gln Lys Gln Phe Ser Thr Leu Val Asp
                485                 490                 495

Ser Thr Ala Thr Ala Gly Lys Lys Ser Pro Ser Ser Phe Gln Phe Asn
            500                 505                 510

Trp Thr Glu Glu Asp Thr Asp Arg Thr Cys Phe His Gly His Ser Leu
        515                 520                 525

Gln Gly Val Leu Lys Glu Lys Gly Gln Ser Leu Leu Thr Lys Asn Ser
    530                 535                 540

Leu Tyr Trp Leu Ser Thr Gln Lys Phe Cys Arg Trp
545                 550                 555


<210> 8
<211> 124
<212> PRT
<213> Homo sapiens

<400> 8
Ile Pro Ala Phe Pro Ala Gly Thr Val Leu Gln Pro Phe Pro Glu Ala
  1               5                  10                  15

Ala Leu Ala Thr Arg Val Thr Val Pro Ala Val Glu Ala Pro Ala Ala
             20                  25                  30

Pro Arg Leu Asp Leu Glu Glu Ser Glu Glu Phe Lys Glu Arg Cys Thr
         35                  40                  45

Gln Cys Ala Ala Val Ser Trp Gly Leu Thr Asp Glu Gly Lys Tyr Tyr
     50                  55                  60

Cys Thr Ser Cys His Asn Val Thr Glu Arg Tyr Gln Glu Val Thr Asn
 65                  70                  75                  80

Thr Asp Leu Ile Pro Asn Thr Gln Ile Lys Ala Leu Asn Arg Gly Leu
                 85                  90                  95

Lys Lys Lys Thr Ile Leu Lys Lys Ala Gly Ile Gly Met Cys Val Lys
            100                 105                 110

Val Ser Ser Ile Phe Phe Ile Asn Lys Gln Lys Pro
        115                 120


<210> 9
<211> 102
<212> PRT
<213> Homo sapiens

<400> 9
Ile Pro Ala Phe Pro Ala Gly Thr Val Leu Gln Pro Phe Pro Glu Ala
  1               5                  10                  15

Ala Leu Ala Thr Arg Val Thr Val Pro Ala Val Glu Ala Pro Ala Ala
             20                  25                  30

Pro Arg Leu Asp Leu Glu Glu Ser Glu Glu Phe Lys Glu Arg Cys Thr
         35                  40                  45

Gln Cys Ala Ala Val Ser Trp Gly Leu Thr Asp Glu Gly Lys Tyr Tyr
     50                  55                  60

Cys Thr Ser Cys His Asn Val Thr Glu Arg Tyr Gln Glu Val Thr Asn
 65                  70                  75                  80

Thr Asp Leu Ile Pro Asn Thr Gln Ile Lys Ala Leu Asn Arg Gly Leu
                 85                  90                  95

Lys Lys Lys Lys Gln Tyr
            100

<210> 10
<211> 343
<212> PRT
<213> Homo sapiens

<400> 10
Met Glu Ser Lys Tyr Lys Glu Ile Leu Leu Leu Thr Gly Leu Asp Asn
1               5                   10                  15

Ile Thr Asp Glu Glu Leu Asp Arg Phe Lys Phe Phe Leu Ser Asp Glu
            20                  25                  30

Phe Asn Ile Ala Thr Gly Lys Leu His Thr Ala Asn Arg Ile Gln Val
        35                  40                  45

Ala Thr Leu Met Ile Gln Asn Ala Gly Ala Val Ser Ala Val Met Lys
    50                  55                  60

Thr Ile Arg Ile Phe Gln Lys Leu Asn Tyr Met Leu Leu Ala Lys Arg
65                  70                  75                  80

Leu Gln Glu Glu Lys Glu Lys Val Asp Lys Gln Tyr Lys Ser Val Thr
                85                  90                  95

Lys Pro Lys Pro Leu Ser Gln Ala Glu Met Ser Pro Ala Ala Ser Ala
            100                 105                 110

Ala Ile Arg Asn Asp Val Ala Lys Gln Arg Ala Ala Pro Lys Val Ser
        115                 120                 125

Pro His Val Lys Pro Glu Gln Lys Gln Met Val Ala Gln Gln Glu Ser
    130                 135                 140

Ile Arg Glu Gly Phe Gln Lys Arg Cys Leu Pro Val Met Val Leu Lys
145                 150                 155                 160

Ala Lys Lys Pro Phe Thr Phe Glu Thr Gln Glu Gly Lys Gln Glu Met
                165                 170                 175

Phe His Ala Thr Val Ala Thr Glu Lys Glu Phe Phe Phe Val Lys Val
            180                 185                 190

Phe Asn Thr Leu Leu Lys Asp Lys Phe Ile Pro Lys Arg Ile Ile Ile
        195                 200                 205

Ile Ala Arg Tyr Tyr Arg His Ser Gly Phe Leu Glu Val Asn Ser Ala
    210                 215                 220

Ser Arg Val Leu Asp Ala Glu Ser Asp Gln Lys Val Asn Val Pro Leu
225                 230                 235                 240

Asn Ile Ile Arg Lys Ala Gly Glu Thr Pro Lys Ile Asn Thr Leu Gln
                245                 250                 255

Thr Gln Pro Leu Gly Thr Ile Val Asn Gly Leu Phe Val Val Gln Lys
            260                 265                 270

Val Thr Glu Lys Lys Lys Asn Ile Leu Phe Asp Leu Ser Asp Asn Thr
        275                 280                 285

Gly Lys Met Glu Val Leu Gly Val Arg Asn Glu Asp Thr Met Lys Cys
    290                 295                 300

Lys Glu Gly Asp Lys Val Arg Leu Thr Phe Phe Thr Leu Ser Lys Asn
305                 310                 315                 320

Gly Glu Lys Leu Gln Leu Thr Ser Gly Val His Ser Thr Ile Lys Val
                325                 330                 335

Ile Lys Ala Lys Lys Lys Thr
            340

<210> 11
<211> 355
<212> PRT
<213> Homo sapiens

<400> 11
Met Glu Ser Lys Tyr Lys Glu Ile Leu Leu Leu Thr Gly Leu Asp Asn
  1               5                  10                  15

Ile Thr Asp Glu Glu Leu Asp Arg Phe Lys Phe Phe Leu Ser Asp Glu
             20                  25                  30

Phe Asn Ile Ala Thr Gly Lys Leu His Thr Ala Asn Arg Ile Gln Val
         35                  40                  45

Ala Thr Leu Met Ile Gln Asn Ala Gly Ala Val Ser Ala Val Met Lys
     50                  55                  60

Thr Ile Arg Ile Phe Gln Lys Leu Asn Tyr Met Leu Leu Ala Lys Arg
 65                  70                  75                  80

Leu Gln Glu Glu Lys Glu Lys Val Asp Lys Gln Tyr Lys Ser Val Thr
                 85                  90                  95

Lys Pro Lys Pro Leu Ser Gln Ala Glu Met Ser Pro Ala Ala Ser Ala
            100                 105                 110

Ala Ile Arg Asn Asp Val Ala Lys Gln Arg Ala Ala Pro Lys Val Ser
        115                 120                 125

Pro His Val Lys Pro Glu Gln Lys Gln Met Val Ala Gln Gln Glu Ser
    130                 135                 140

Ile Arg Glu Gly Phe Gln Lys Arg Cys Leu Pro Val Met Val Leu Lys
145                 150                 155                 160

Ala Lys Lys Pro Phe Thr Phe Glu Thr Gln Glu Gly Lys Gln Glu Met
                165                 170                 175

Phe His Ala Thr Val Ala Thr Glu Lys Glu Phe Phe Phe Val Lys Val
            180                 185                 190

Phe Asn Thr Leu Leu Lys Asp Lys Phe Ile Pro Lys Arg Ile Ile Ile
        195                 200                 205

Ile Ala Arg Tyr Tyr Arg His Ser Gly Phe Leu Glu Val Asn Ser Ala
    210                 215                 220

Ser Arg Val Leu Asp Ala Glu Ser Asp Gln Lys Val Asn Val Pro Leu
225                 230                 235                 240

Asn Ile Ile Arg Lys Ala Gly Glu Thr Pro Lys Ile Asn Thr Leu Gln
                245                 250                 255

Thr Gln Pro Leu Gly Thr Ile Val Asn Gly Leu Phe Val Val Gln Lys
            260                 265                 270

Val Thr Glu Lys Lys Lys Asn Ile Leu Phe Asp Leu Ser Asp Asn Thr
        275                 280                 285

Gly Lys Met Glu Val Leu Gly Val Arg Asn Glu Asp Thr Met Lys Cys
    290                 295                 300

Lys Glu Gly Asp Lys Val Arg Leu Thr Phe Phe Thr Leu Ser Lys Asn
305                 310                 315                 320

Gly Glu Lys Leu Gln Leu Thr Ser Gly Val His Ser Thr Ile Lys Val
                325                 330                 335

Ile Lys Ala Lys Lys Lys His Arg Glu Val Lys Arg Thr Asn Ser Ser
            340                 345                 350

Gln Leu Val
        355


<210> 12
<211> 807
<212> PRT
<213> Homo sapiens

<400> 12
Met Pro Lys Ala Pro Lys Gln Gln Pro Pro Glu Pro Glu Trp Ile Gly
  1               5                  10                  15

Asp Gly Glu Ser Thr Ser Pro Ser Asp Lys Val Val Lys Lys Gly Lys
             20                  25                  30

Lys Asp Lys Lys Ile Lys Lys Thr Phe Phe Glu Glu Leu Ala Val Glu
         35                  40                  45

Asp Lys Gln Ala Gly Glu Glu Glu Lys Val Leu Lys Glu Lys Glu Gln
     50                  55                  60

Gln Gln Gln Gln Gln Gln Gln Gln Gln Lys Lys Lys Arg Asp Thr Arg
 65                  70                  75                  80

Lys Gly Arg Arg Lys Lys Asp Val Asp Asp Asp Gly Glu Glu Lys Glu
                 85                  90                  95

Leu Met Glu Arg Leu Lys Lys Leu Ser Val Pro Thr Ser Asp Glu Glu
            100                 105                 110

Asp Glu Val Pro Ala Pro Lys Pro Arg Gly Gly Lys Lys Thr Lys Gly
        115                 120                 125

Gly Asn Val Phe Ala Ala Leu Ile Gln Asp Gln Ser Glu Glu Glu Glu
    130                 135                 140

Glu Glu Glu Lys His Pro Pro Lys Pro Ala Lys Pro Glu Lys Asn Arg
145                 150                 155                 160

Ile Asn Lys Ala Val Ser Glu Glu Gln Gln Pro Ala Leu Lys Gly Lys
                165                 170                 175

Lys Gly Lys Glu Glu Lys Ser Lys Gly Lys Ala Lys Pro Gln Asn Lys
            180                 185                 190

Phe Ala Ala Leu Asp Asn Glu Glu Glu Asp Lys Glu Glu Glu Ile Ile
        195                 200                 205

Lys Glu Lys Glu Pro Pro Lys Gln Gly Lys Glu Lys Ala Lys Lys Ala
    210                 215                 220

Glu Gln Met Glu Tyr Glu Arg Gln Val Ala Ser Leu Lys Ala Ala Asn
225                 230                 235                 240

Ala Ala Glu Asn Asp Phe Ser Val Ser Gln Ala Glu Met Ser Ser Arg
                245                 250                 255

Gln Ala Met Leu Glu Asn Ala Ser Asp Ile Lys Leu Glu Lys Phe Ser
            260                 265                 270

Ile Ser Ala His Gly Lys Glu Leu Phe Val Asn Ala Asp Leu Tyr Ile
        275                 280                 285

Val Ala Gly Arg Arg Tyr Gly Leu Val Gly Pro Asn Gly Lys Gly Lys
    290                 295                 300

Thr Thr Leu Leu Lys His Ile Ala Asn Arg Ala Leu Ser Ile Pro Pro
305                 310                 315                 320

Asn Ile Asp Val Leu Leu Cys Glu Gln Glu Val Val Ala Asp Glu Thr
                325                 330                 335

Pro Ala Val Gln Ala Val Leu Arg Ala Asp Thr Lys Arg Leu Lys Leu
            340                 345                 350

Leu Glu Glu Glu Arg Arg Leu Gln Gly Gln Leu Glu Gln Gly Asp Asp
        355                 360                 365

Thr Ala Ala Glu Arg Leu Glu Lys Val Tyr Glu Glu Leu Arg Ala Thr
    370                 375                 380

Gly Ala Ala Ala Ala Glu Ala Lys Ala Arg Arg Ile Leu Ala Gly Leu
385                 390                 395                 400

Gly Phe Asp Pro Glu Met Gln Asn Arg Pro Thr Gln Lys Phe Ser Gly
                405                 410                 415

Gly Trp Arg Met Arg Val Ser Leu Ala Arg Ala Leu Phe Met Glu Pro
            420                 425                 430

Thr Leu Leu Met Leu Asp Glu Pro Thr Asn His Leu Asp Leu Asn Ala
        435                 440                 445

Val Ile Trp Leu Asn Asn Tyr Leu Gln Gly Trp Arg Lys Thr Leu Leu
    450                 455                 460

Ile Val Ser His Asp Gln Gly Phe Leu Asp Asp Val Cys Thr Asp Ile
465                 470                 475                 480

Ile His Leu Asp Ala Gln Arg Leu His Tyr Tyr Arg Gly Asn Tyr Met
                485                 490                 495

Thr Phe Lys Lys Met Tyr Gln Gln Lys Gln Lys Glu Leu Leu Lys Gln
            500                 505                 510

Tyr Glu Lys Gln Glu Lys Lys Leu Lys Glu Leu Lys Ala Gly Gly Lys
        515                 520                 525

Ser Thr Lys Gln Ala Glu Lys Gln Thr Lys Glu Ala Leu Thr Arg Lys
    530                 535                 540

Gln Gln Lys Cys Arg Arg Lys Asn Gln Asp Glu Glu Ser Gln Glu Ala
545                 550                 555                 560

Pro Glu Leu Leu Lys Arg Pro Lys Glu Tyr Thr Val Arg Phe Thr Phe
                565                 570                 575

Pro Asp Pro Pro Pro Leu Ser Pro Pro Val Leu Gly Leu His Gly Val
            580                 585                 590

Thr Phe Gly Tyr Gln Gly Gln Lys Pro Leu Phe Lys Asn Leu Asp Phe
        595                 600                 605

Gly Ile Asp Met Asp Ser Arg Ile Cys Ile Val Gly Pro Asn Gly Val
    610                 615                 620

Gly Lys Ser Thr Leu Leu Leu Leu Leu Thr Gly Lys Leu Thr Pro Thr
625                 630                 635                 640

His Gly Glu Met Arg Lys Asn His Arg Leu Lys Ile Gly Phe Phe Asn
                645                 650                 655

Gln Gln Tyr Ala Glu Gln Leu Arg Met Glu Glu Thr Pro Thr Glu Tyr
            660                 665                 670

Leu Gln Arg Gly Phe Asn Leu Pro Tyr Gln Asp Ala Arg Lys Cys Leu
        675                 680                 685

Gly Arg Phe Gly Leu Glu Ser His Ala His Thr Ile Gln Ile Cys Lys
    690                 695                 700

Leu Ser Gly Gly Gln Lys Ala Arg Val Val Phe Ala Glu Leu Ala Cys
705                 710                 715                 720

Arg Glu Pro Asp Val Leu Ile Leu Asp Glu Pro Thr Asn Asn Leu Asp
                725                 730                 735

Ile Glu Ser Ile Asp Ala Leu Gly Glu Ala Ile Asn Glu Tyr Lys Gly
            740                 745                 750

Ala Val Ile Val Val Ser His Asp Ala Arg Leu Ile Thr Glu Thr Asn
        755                 760                 765

Cys Gln Leu Trp Val Val Glu Glu Gln Ser Val Ser Gln Ile Asp Gly
    770                 775                 780

Asp Phe Glu Asp Tyr Lys Arg Glu Val Leu Glu Ala Leu Gly Glu Val
785                 790                 795                 800

Met Val Ser Arg Pro Arg Glu
                805


<210> 13
<211> 134
<212> PRT
<213> Homo sapiens

<400> 13
Met Pro Lys Ala Pro Lys Gln Gln Pro Pro Glu Pro Glu Trp Ile Gly
  1               5                  10                  15

Asp Gly Glu Ser Thr Ser Pro Ser Asp Lys Val Val Lys Lys Gly Lys
             20                  25                  30

Lys Asp Lys Lys Ile Lys Lys Thr Phe Phe Glu Glu Leu Ala Val Glu
         35                  40                  45

Asp Lys Gln Ala Gly Glu Glu Glu Lys Val Leu Lys Glu Lys Glu Gln
     50                  55                  60

Gln Gln Gln Gln Gln Gln Gln Gln Gln Lys Lys Ser Glu Ile Pro Glu
 65                  70                  75                  80

Lys Ala Gly Gly Arg Arg Met Trp Met Met Met Glu Lys Arg Lys Ser
                 85                  90                  95

Ser Trp Ser Val Leu Arg Ser Ser Gln Cys Gln Pro Val Met Arg Arg
            100                 105                 110

Met Lys Tyr Pro Pro Gln Asn Pro Ala Glu Gly Arg Lys Pro Arg Val
        115                 120                 125

Val Met Phe Leu Gln Pro
    130


<210> 14
<211> 89
<212> PRT
<213> Homo sapiens

<400> 14
Met Pro Lys Ala Pro Lys Gln Gln Pro Pro Glu Pro Glu Trp Ile Gly
1               5                   10                  15

Asp Gly Glu Ser Thr Ser Pro Ser Asp Lys Val Val Lys Lys Gly Lys
            20                  25                  30

Lys Asp Lys Lys Ile Lys Lys Thr Phe Phe Glu Glu Leu Ala Val Glu
        35                  40                  45

Asp Lys Gln Ala Gly Glu Glu Glu Lys Val Leu Lys Glu Lys Glu Gln
    50                  55                  60

Gln Gln Gln Gln Gln Gln Gln Gln Gln Lys Lys Lys Ala Arg Tyr Pro
65                  70                  75                  80

Lys Arg Gln Ala Glu Glu Gly Cys Gly
                85


<210> 15
<211> 246
<212> PRT
<213> Homo sapiens

<400> 15

<210> 16
<211> 148
<212> PRT
<213> Homo sapiens

<400> 16
Met Ala Phe Leu Arg Ser Met Trp Gly Val Leu Ser Ala Leu Gly Arg
1               5                   10                  15

Ser Gly Ala Glu Leu Cys Thr Gly Cys Gly Ser Arg Leu Arg Ser Pro
            20                  25                  30

Phe Ser Phe Val Tyr Leu Pro Arg Trp Phe Ser Ser Val Leu Ala Ser
        35                  40                  45

Cys Pro Lys Lys Pro Val Ser Ser Tyr Leu Arg Phe Ser Lys Glu Gln
    50                  55                  60

Leu Pro Ile Phe Lys Ala Gln Asn Pro Asp Ala Lys Thr Thr Glu Leu
65                  70                  75                  80

Ile Arg Arg Ile Ala Gln Arg Trp Arg Glu Leu Pro Asp Ser Lys Lys
                85                  90                  95

Lys Ile Tyr Gln Asp Ala Tyr Arg Ala Glu Trp Gln Val Tyr Lys Glu
            100                 105                 110

Glu Ile Ser Arg Phe Lys Glu Gln Leu Thr Pro Ser Gln Ile Met Ser
        115                 120                 125

Leu Glu Lys Glu Ile Met Asp Lys His Leu Lys Arg Lys Ala Met Thr
    130                 135                 140

Lys Lys Lys Ser
145

<210> 17
<211> 162
<212> PRT
<213> Homo sapiens

<400> 17
Met Ala Phe Leu Arg Ser Met Trp Gly Val Leu Ser Ala Leu Gly Arg
1               5                   10                  15

Ser Gly Ala Glu Leu Cys Thr Gly Cys Gly Ser Arg Leu Arg Ser Pro
                20                  25                  30

Phe Ser Phe Val Tyr Leu Pro Arg Trp Phe Ser Ser Val Leu Ala Ser
            35                  40                  45

Cys Pro Lys Lys Pro Val Ser Ser Tyr Leu Arg Phe Ser Lys Glu Gln
    50                  55                  60

Leu Pro Ile Phe Lys Ala Gln Asn Pro Asp Ala Lys Thr Thr Glu Leu
65                  70                  75                  80

Ile Arg Arg Ile Ala Gln Arg Trp Arg Glu Leu Pro Asp Ser Lys Lys
                85                  90                  95

Lys Ile Tyr Gln Asp Ala Tyr Arg Ala Glu Trp Gln Val Tyr Lys Glu
            100                 105                 110

Glu Ile Ser Arg Phe Lys Glu Gln Leu Thr Pro Ser Gln Ile Met Ser
        115                 120                 125

Leu Glu Lys Glu Ile Met Asp Lys His Leu Lys Arg Lys Ala Met Thr
    130                 135                 140

Lys Lys Lys Arg Val Asn Thr Ala Trp Lys Thr Lys Lys Thr Ser Phe
145                 150                 155                 160

Ser Leu

<210> 18
<211> 350
<212> PRT
<213> Homo sapiens

<400> 18


<210> 19
<211> 40
<212> PRT
<213> Homo sapiens

<400> 19
Met Glu Ser Gln Val Gly Gly Ala Arg Pro Ala Gly Leu Pro Asn Gly
1               5                   10                  15

His Ser Leu Val Gln Met Glu Pro Leu Thr Thr Ala Arg Pro Thr Ser
            20                  25                  30

Ser Ser Thr Thr Cys Pro Arg Thr
        35                  40


<210> 20
<211> 25
<212> PRT
<213> Homo sapiens

<400> 20
Met Glu Ser Gln Val Gly Gly Gly Pro Gly Arg Pro Ala Cys Pro Thr
1               5                   10                  15

Ala Thr Pro Trp Tyr Lys Trp Ser His
            20                  25


<210> 21

<211> 648
<212> PRT
<213> Homo sapiens

<400> 21
Met Ser Ala Ser Ala Ser Val Gly Gly Pro Val Pro Gln Pro Pro Pro
1               5                   10                  15

Gly Pro Ala Ala Ala Leu Pro Pro Gly Ser Ala Ala Arg Ala Leu His
            20                  25                  30

Val Glu Leu Pro Ser Gln Gln Arg Arg Leu Arg His Leu Arg Asn Ile
        35                  40                  45

Ala Ala Arg Asn Ile Val Asn Arg Asn Gly His Gln Leu Leu Asp Thr
    50                  55                  60

Tyr Phe Thr Leu His Leu Cys Ser Thr Glu Lys Ile Tyr Lys Glu Phe
65                  70                  75                  80

Tyr Arg Ser Glu Val Ile Lys Asn Ser Leu Asn Pro Thr Trp Arg Ser
                85                  90                  95

Leu Asp Phe Gly Ile Met Pro Asp Arg Leu Asp Thr Ser Val Ser Cys
            100                 105                 110

Phe Val Val Lys Ile Trp Gly Gly Lys Glu Asn Ile Tyr Gln Leu Leu
        115                 120                 125

Ile Glu Trp Lys Val Cys Leu Asp Gly Leu Lys Tyr Leu Gly Gln Gln
    130                 135                 140

Ile His Ala Arg Asn Gln Asn Glu Ile Ile Phe Gly Leu Asn Asp Gly
145                 150                 155                 160

Tyr Tyr Gly Ala Pro Phe Glu His Lys Gly Tyr Ser Asn Ala Gln Lys
                165                 170                 175

Thr Ile Leu Leu Gln Val Asp Gln Asn Cys Val Arg Asn Ser Tyr Asp
            180                 185                 190

Val Phe Ser Leu Leu Arg Leu His Arg Ala Gln Cys Ala Ile Lys Gln
        195                 200                 205

Thr Gln Val Thr Val Gln Lys Ile Gly Lys Glu Ile Glu Glu Lys Leu
    210                 215                 220

Arg Leu Thr Ser Thr Ser Asn Glu Leu Lys Lys Lys Ser Glu Cys Leu
225                 230                 235                 240

Gln Leu Lys Ile Leu Val Leu Gln Asn Glu Leu Glu Arg Gln Lys Lys
                245                 250                 255

Ala Leu Gly Arg Glu Val Ala Leu Leu His Lys Gln Gln Ile Ala Leu
            260                 265                 270

Gln Asp Lys Gly Ser Ala Phe Ser Ala Glu His Leu Lys Leu Gln Leu
        275                 280                 285

Gln Lys Glu Ser Leu Asn Glu Leu Arg Lys Glu Cys Thr Ala Lys Arg
    290                 295                 300

Glu Leu Phe Leu Lys Thr Asn Ala Gln Leu Thr Ile Arg Cys Arg Gln
305                 310                 315                 320

Leu Leu Ser Glu Leu Ser Tyr Ile Tyr Pro Ile Asp Leu Asn Glu His
                325                 330                 335

Lys Asp Tyr Phe Val Cys Gly Val Lys Leu Pro Asn Ser Glu Asp Phe
            340                 345                 350

Gln Ala Lys Asp Asp Gly Ser Ile Ala Val Ala Leu Gly Tyr Thr Ala
        355                 360                 365

His Leu Val Ser Met Ile Ser Phe Phe Leu Gln Val Pro Leu Arg Tyr
    370                 375                 380

Pro Ile Ile His Lys Gly Ser Arg Ser Thr Ile Lys Asp Asn Ile Asn
385                 390                 395                 400

Asp Lys Leu Thr Glu Lys Glu Arg Glu Phe Pro Leu Tyr Pro Lys Gly
                405                 410                 415

Gly Glu Lys Leu Gln Phe Asp Tyr Gly Val Tyr Leu Leu Asn Lys Asn
            420                 425                 430

Ile Ala Gln Leu Arg Tyr Gln His Gly Leu Gly Thr Pro Asp Leu Arg
        435                 440                 445

Gln Thr Leu Pro Asn Leu Lys Asn Phe Met Glu His Gly Leu Met Val
    450                 455                 460

Arg Cys Asp Arg His His Thr Ser Ser Ala Ile Pro Val Pro Lys Arg
465                 470                 475                 480

Gln Ser Ser Ile Phe Gly Gly Ala Asp Val Gly Phe Ser Gly Gly Ile
                485                 490                 495

Pro Ser Pro Asp Lys Gly His Arg Lys Arg Ala Ser Ser Glu Asn Glu
            500                 505                 510

Arg Leu Gln Tyr Lys Thr Pro Pro Pro Ser Tyr Asn Ser Ala Leu Ala
        515                 520                 525

Gln Pro Val Thr Thr Val Pro Ser Met Gly Glu Thr Glu Arg Lys Ile
    530                 535                 540

Thr Ser Leu Ser Ser Ser Leu Asp Thr Ser Leu Asp Phe Ser Lys Glu
545                 550                 555                 560

Asn Lys Lys Lys Gly Glu Asp Leu Val Gly Ser Leu Asn Gly Gly His
                565                 570                 575

Ala Asn Val His Pro Ser Gln Glu Gln Gly Glu Ala Leu Ser Gly His
            580                 585                 590

Arg Ala Thr Val Asn Gly Thr Leu Leu Pro Ser Glu Gln Ala Gly Ser
        595                 600                 605

Ala Ser Val Gln Leu Pro Gly Glu Phe His Pro Val Ser Glu Ala Glu
    610                 615                 620

Leu Cys Cys Thr Val Glu Gln Ala Glu Glu Ile Ile Gly Leu Glu Ala
625                 630                 635                 640

Gln Val Ser Pro Gln Val Ile Ser
                645

<210> 22
<211> 241
<212> PRT
<213> Homo sapiens

<400> 22

Met Ser Ala Ser Ala Ser Val Gly Gly Pro Val Pro Gln Pro Pro Pro
1               5                   10                  15

Gly Pro Ala Ala Ala Leu Pro Pro Gly Ser Ala Ala Arg Ala Leu His
            20                  25                  30

Val Glu Leu Pro Ser Gln Gln Arg Arg Leu Arg His Leu Arg Asn Ile
        35                  40                  45

Ala Ala Arg Asn Ile Val Asn Arg Asn Gly His Gln Leu Leu Asp Thr
    50                  55                  60

Tyr Phe Thr Leu His Leu Cys Ser Thr Glu Lys Ile Tyr Lys Glu Phe
65                  70                  75                  80

Tyr Arg Ser Glu Val Ile Lys Asn Ser Leu Asn Pro Thr Trp Arg Ser
                85                  90                  95

Leu Asp Phe Gly Ile Met Pro Asp Arg Leu Asp Thr Ser Val Ser Cys
            100                 105                 110

Phe Val Val Lys Ile Trp Gly Gly Lys Glu Asn Ile Tyr Gln Leu Leu
        115                 120                 125

Ile Glu Trp Lys Val Cys Leu Asp Gly Leu Lys Tyr Leu Gly Gln Gln
    130                 135                 140

Ile His Ala Arg Asn Gln Asn Glu Ile Ile Phe Gly Leu Asn Asp Gly
145                 150                 155                 160

Tyr Tyr Gly Ala Pro Phe Glu His Lys Gly Tyr Ser Asn Ala Gln Lys
                165                 170                 175

Thr Ile Leu Leu Gln Val Asp Gln Asn Cys Val Arg Asn Ser Tyr Asp
            180                 185                 190

Val Phe Ser Leu Leu Arg Leu His Arg Ala Gln Cys Ala Ile Lys Gln
        195                 200                 205

Thr Gln Val Thr Val Gln Lys Ile Gly Lys Glu Ile Glu Glu Lys Leu
    210                 215                 220

Arg Leu Thr Ser Thr Ser Asn Glu Leu Lys Lys Lys Val Asn Ala Cys
225                 230                 235                 240

Ser

<210> 23
<211> 237
<212> PRT
<213> Homo sapiens

<400> 23